# EUROPEAN PATENT APPLICATION

(11) **EP 4 001 411 A1**
(43) Date of publication of application: **25.05.2022**
(21) Application number: 20306419.1
(22) Date of filing: 20.11.2020
(51) Int. Cl.: C12N 15/11

(54) **ISOLATED DOUBLE STRANDED DNA POLYNUCLEOTIDE**

(71) Applicant: Université de Lorraine, 54052 Nancy Cedex (FR); Centre national de la recherche scientifique, 75016 Paris (FR)
(72) Inventor: Maenner, Sylvain, 54000 Nancy (FR); Behm-Ansmant, Isabelle, 54800 Brainville (FR); Alfeghaly, Charbel, 75013 Paris (FR)
(74) Representative: Novagraaf Technologies

(57) **Abstract**

The present invention relates to an isolated double stranded DNA polynucleotide that forms triplex with sequence 5'-GGUGGCAGCAAGAGAAAAAUGAGGAAGAAGCAAAAGCGGAAA-3' (SEQ ID NO: 1) of the long non-coding RNA ANRIL (Antisense Non-coding RNA in the INK4 Locus).

It also relates to a vector comprising the double stranded DNA polynucleotide, and to a pharmaceutical composition comprising the double stranded DNA polynucleotide or the vector.

The present invention relates as well to the isolated double stranded DNA polynucleotide for use in the treatment of myocardial infarction, aneurysms, stenosis, myocardial infarction, aneurysms, cancers, eye diseases or type 2 diabetes.

SEQUENCE LISTING

<110>  UNIVERSITE DE LORRAINE
       CNRS

<120>  ISOLATED DOUBLE STRANDED DNA POLYNUCLEOTIDE

<130>  BNT229739EP00

<160>  24

<170>  PatentIn version 3.5

<210>  1
<211>  42
<212>  RNA
<213>  Homo sapiens

<400>  1
gguggcagca agagaaaaau gaggaagaag caaaagcgga aa                          42


<210>  2
<211>  42
<212>  DNA
<213>  Unknown

<220>
<223>  artificial sequence

<400>  2
aaaggcgaaa acgaagaagg agtaaaaaga gaacgacggt gg                          42


<210>  3
<211>  42
<212>  DNA
<213>  Unknown

<220>
<223>  artificial sequence

<400>  3
ccaccgtcgt tctcttttta ctccttcttc gttttcgcct tt                          42


<210>  4
<211>  42
<212>  DNA
<213>  Unknown

<220>
<223>  artificial sequence

<400>  4
aaaggggaaa aggaagaagg agaaaaaaga gaaggaggga gg                          42


<210>  5
<211>  42
<212>  DNA
<213>  Unknown

<220>
<223>  artificial sequence

<400>  5
cctccctcct tctctttttt ctccttcttc cttttcccct tt                    42


<210>  6
<211>  25
<212>  DNA
<213>  Unknown

<220>
<223>  primer

<400>  6
caccgatatc agtgaaggcg ttcat                                      25


<210>  7
<211>  25
<212>  DNA
<213>  Unknown

<220>
<223>  primer

<400>  7
aaacatgaac gccttcactg atatc                                      25


<210>  8
<211>  25
<212>  DNA
<213>  Unknown

<220>
<223>  primer

<400>  8
caccgaccca gagggaggta aatta                                      25


<210>  9
<211>  25
<212>  DNA
<213>  Unknown

<220>
<223>  primer

<400>  9
aaactaattt acctccctct gggtc                                      25


<210>  10
<211>  16
<212>  DNA
<213>  Unknown

<220>
<223>  artificial sequence

<400>  10
gctcccttca atccaa                                                       16


<210>  11
<211>  16
<212>  DNA
<213>  Unknown

<220>
<223>  artificial sequence

<400>  11
tcagaggcgt gcagcg                                                       16


<210>  12
<211>  16
<212>  DNA
<213>  Unknown

<220>
<223>  artificial sequence

<400>  12
taagatccag tggtgg                                                       16


<210>  13
<211>  16
<212>  DNA
<213>  Unknown

<220>
<223>  artificial sequence

<400>  13
cgtaatcatc catgca                                                       16


<210>  14
<211>  15
<212>  DNA
<213>  Unknown

<220>
<223>  artificial sequence

<400>  14
aatcatcctg tcaaa                                                        15


<210>  15
<211>  696
<212>  RNA
<213>  Homo sapiens

<400>  15
ucucuccaau gaacgccuuc acugauaucc aaagcaugaa ggacacacca gggaaaaaca      60

uagaccuaac acaggacaaa uggaauuauu agaaacauuu ucuagcagaa gaacacuauu     120

cuguugccau uugaaucuuu gcuucuuucu agguuugaca augagccuau cauauaagcc     180

caaauguaaa cagaaagagg uugaaucagu cacgauaagc ccaauuaugc ugugguaaca 240

aacaaccuca aaaucucauu ggcuuaaaau auacagaauu auucuuacuc auggcacaua 300

uccaucuauc aucugcaggg gaucugcuca cugaagucac uuaggaacuu ggacugaugg 360

aacggccacu uuuuggucac uauauguauu aaucuguuuu aauccugcug auaaagaccc 420

aaaauuggga acaaaaagaa guuuaacuag acuuacaguu ccgcauggcu gaggaggccu 480

cagaaucaug gugggaggcg aaaggcacuu cuuacauggu ggcagcaaga gaaaaaugag 540

gaagaagcaa aagcggaaac cucugauaaa cccaucagau cuuaugagac uuauuccacu 600

aucaagagaa uagcauggga aagacuggcu cccauaauuu accucccucu gggucccucc 660

cucaacaugu gggaauucug ggagaaacaa uucaag 696

<210> 16
<211> 15
<212> RNA
<213> Homo sapiens

<400> 16
aggaagaagc aaaag 15

<210> 17
<211> 15
<212> DNA
<213> Unknown

<220>
<223> artificial sequence

<400> 17
gaagagggag aagga 15

<210> 18
<211> 15
<212> DNA
<213> Unknown

<220>
<223> artificial sequence

<400> 18
tccttctccc tcttc 15

<210> 19
<211> 21
<212> RNA
<213> Unknown

<220>
<223> artificial sequence

<400> 19
ggaaggcagg gagagguaga a 21

<210>  20
<211>  21
<212>  DNA
<213>  Unknown

<220>
<223>  artificial sequence

<400>  20
gagagagaga gggaaggaag g                                                21


<210>  21
<211>  21
<212>  DNA
<213>  Unknown

<220>
<223>  artificial sequence

<400>  21
ccttccttcc ctctctctct c                                                21


<210>  22
<211>  14
<212>  RNA
<213>  Homo sapiens

<400>  22
gaggaagaag caaa                                                        14


<210>  23
<211>  15
<212>  DNA
<213>  Unknown

<220>
<223>  artificial sequence

<400>  23
ctcgttcttc tattt                                                       15


<210>  24
<211>  15
<212>  DNA
<213>  Unknown

<220>
<223>  artificial sequence

<400>  24
aaatagaaga acgag                                                       15

## Description

### Technical field

The present invention refers to an isolated double stranded DNA polynucleotide that forms triplex with a sequence of the long non-coding RNA ANRIL (Antisense Non-coding RNA in the INK4 Locus).

Therefore, the present invention has utility in medical fields.

In the description below, the references into brackets ([]) refer to the listing of references situated at the end of the text.

### Background of the Invention

In humans, multiple pathologies are due to inappropriate regulation of gene expression. The development of strategies aimed at restoring normal gene expression is therefore a major public health issue. These so-called "gene" therapies are complex and require several scientific obstacles to be overcome. One of them lies in the specificity of the therapeutic agent's action. Without specificity, the latter can cause a cascade of undesirable effects.

Non-coding RNAs do not have coding potential, i.e. they are not translated into proteins (e.g. ribosomal or transfer RNAs involved in the translation process of messenger RNAs).

Over the last decade, next generation sequencing approaches highlighted the unexpected diversity of RNAs lacking obvious protein-coding capacity (ncRNAs). The ncRNAs longer than 200-nts are named long non-coding RNAs (IncRNAs). Nowadays, more than 167,000 IncRNAs have been identified in human, many of them being involved in various critical processes including cell proliferation and cell differenciation. The deregulation of the expression of these IncRNAs can therefore affect cell homeostasis and consequently favour the occurrence and/or development of pathologies. They can then be qualified as pathogenic IncRNAs.

According to "the IncRNADisease database", IncRNAs are associated with 529 pathologies divided into several categories, including 3 major ones corresponding to cancers (44.2%), cardiovascular pathologies (11.6%) and neurodegenerative diseases (7.3%). Several IncRNAs are already used as biomarkers as their expression rate correlates with the diagnostic or even pronostic nature of certain pathologies. It is the case of IncRNA PCA3, used as a pronostic biomarker for prostate cancer.

Within the cell, IncRNAs can be located either in the cytoplasm or in the nucleus.

LncRNAs are key regulators of gene expression carrying both cytoplasmic and nuclear functions. Cytoplasmic IncRNAs mainly modulate gene expression by affecting mRNA stability or translation, while nuclear IncRNAs are mostly associate with the genome to regulate gene expression at the chromatin level. The latter implies the activities of epigenetic writers to targeted genomic loci, including for instance the Polycomb group proteins (PcG) composed by the Polycomb repressive complexes 1 and 2 (PRC1 and PRC2). These complexes are responsible for the conversion of euchromatin into heterochromatin by catalyzing the ubiquitylation of histone H2A on lysine 119 (H2AK119Ub) and the trimethylation of histone H3 on lysine 27 (H3K27me3), respectively. Interestingly, multiple IncRNAs have been shown to associate with the PcG and 20% of them are specific PRC2-binders in human cells.

In these gene regulatory mechanisms, nuclear IncRNAs must first recognise and specifically bind the DNA regions expression of which they regulate. To date, this step remains relatively undocumented. The two most widely described modes consist 1/ in the formation of a particular structure called R-loops involving the formation of canonical base pairs between the ncRNA and the DNA molecule and 2/ in the intervention of a DRBP (Doubled stranded RNA Binding Protein) capable of establishing a bridge between the DNA molecule and the IncRNA.

A third mode of interaction involves the formation of particular structures called triplexes [DNA/DNA:RNA]. These triplexes are non-canonical structures in which a single-stranded RNA (Triplex Forming Oligonucleotide, TFO) accommodates the major groove of the DNA double helix (Triplex Targeting Site, TTS). TTSs correspond exclusively to purine-rich sequences (Adenine-A/Guanine-G) and form non-canonical Hoogsteen or reverse Hoogsteen base pairs with TFO (Rajagopal P., and J. Feigon. "Triple-Strand Formation in the Homopurine:homopyrimidine DNA Oligonucleotides d(G-A)4 and d(T-C)4." Nature 339, no. 6226 (June 22, 1989): 637-40 ([1])). According to *in silico* analyses, millions of TTSs have been identified within the mammalian genome and located in regulatory regions such as promoters. The specificity and robustness of the TFO/TTS triplex correlate positively with the GC percentage of TFO and the number of Hoogsteen and inverse Hoogsteen base pairs forming between TFO and TTS (Maldonado et al.: "Purine- and Pyrimidine-Triple-Helix-Forming Oligonucleotides Recognize Qualitatively Different Target Sites at the Ribosomal DNA Locus." RNA (New York, N.Y.) 24, no. 3 (2018): 371-80 ([2])). Historically, triplexes [DNA/DNA:RNA] have been functionally associated with events of transcription, gene silencing and conversion, cell proliferation and double-stranded DNA breaks. Interestingly, several studies suggest that these structures may be also widely used by IncRNAs. Indeed, the formation of triplexes would allow IncRNAs to anchor themselves to chromatin and recruit protein complexes at the gene regions the expression of which they regulate.

ANRIL (Antisense Noncoding RNA in the INK4 Locus) is one of the IncRNAs associated with PcG activities and several pathologies. It is transcribed from the *9p21* locus in the opposite direction to the *CDKN2A* and *CDKN2B* (cyclin dependent kinase inhibitors 2A and 2B) genes. ANRIL promotes *in cis* the transcriptional silencing of the *CDKN2A* and *B* genes by recruiting the PcG to the *9p21* locus resulting in an increased cell proliferation rate. In addition, ANRIL is expected to modulate *in trans* the expression of genes distant from the *9p21* locus. This is evidenced by the differential expression of more than 200 genes involved in the maintenance of chromatin architecture, cellular proliferation, growth and apoptosis upon the overexpression of ANRIL sub-fragments in HeLa or in HEK293 cells. Expression changes were also observed for 20 genes, implicated in proliferation and apoptosis, upon ANRIL siRNA knockdown in vascular smooth muscle cells (VSMCs). Even though previous studies demonstrated the *trans*-regulatory activity of ANRIL, the molecular mechanisms involved need to be refined. Sor far, the coding and non-coding genes, which are directly contacted and regulated by ANRIL remain unknown. Also, the mechanisms engaged by ANRIL to specifically associate with the genome remain to be deciphered.

Thus, a need exists of alternative tools able to modulate the regulatory functions on gene expression of the IncRNAs. The present invention fulfills these and other needs.

### Description of the invention

The present invention describes an original system aimed at finely modulating gene expression by affecting the genomic recognition of the long non-coding RNA called ANRIL.

The Applicants have deployed a strategy used to identify for the first time the genes outside the 9p21 locus directly regulated by ANRIL. For this purpose, they carried out chromatin immunoprecipitation experiments by RNA selection (ChIRP) allowing them to identify at high resolution the genomic loci physically contacted by ANRIL. These ChIRP analyses have enabled them to show that ANRIL contacts 3227 gene regions in human embryonic kidney cells (HEK293).

Surprisingly, the Applicants identify 1477 and 1144 genes with increased or decreased expression respectively upon ANRIL knock-down. Then, the Applicants focused on the 1477 genes whose expression was increased in the absence of ANRIL. They surprisingly identified 123 genes that were physically contacted and negatively regulated by ANRIL, and that correspond to ANRIL's primary gene targets. After extensive research, the Applicants then identified a region within exon 8 of ANRIL (DBD-Ex8 for DNA Binding Domain-Exon8) capable of forming triplexes with 422 regions, and interestingly showed that 23 of them correspond to primary targets. It should be noted that among these 23 genes, several have been linked to pathologies related to ANRIL: cancers and vasculo/oculopathies. These results were therefore consistent with ANRIL's regulatory activities on genes whose expression is deregulated in pathological conditions.

As mentioned above, pathogenic IncRNAs play multiple functions in gene regulation and are associated with the occurrence of diseases. ANRIL is a representative example:
- Under physiological conditions, its expression rate is normal. ANRIL modulates the expression of several primary target genes including those located within the 9p21 locus such as CDKN2A and B genes.
- In pathological conditions, the Applicants surprisingly hypothesize that ANRIL over-expression generates excess ANRIL molecules capable of reinforcing the regulatory activity it exerts on its primary targets or even on additional genomic regions (pathological dysfunction). The molecular aberrations thus generated could be responsible for the appearance and/or reinforcement of pathological processes such as cancers or cardiovascular diseases.

The Applicants thus created a competitive molecule of the double-stranded DNA oligonucleotide type, designed to specifically block the formation of triplexes between genomic DNA and ANRIL. We have named the latter ANRIL-TDO (ANRIL Triplex Decoy Oligonucleotide). Surprisingly, in its presence, ANRIL would be unable to bind to certain genomic loci via triplex formation and therefore unable to exercise its gene regulatory activity observed in pathological conditions.

ANRIL-TDO of the invention has many advantages:
- It specifically modulates the triplex-mediated recognition established by ANRIL within the genome. It therefore offers the advantage of finely and selectively impacting the genes whose expression is regulated by ANRIL in pathological conditions.
- It offers the advantage of targeting ANRIL without affecting its stability or expression. ANRIL is therefore likely to carry out its observed activity under normal conditions. Indeed, ANRIL-TDO is designed to modulate ANRIL activity and not its stability/expression. Without wishing to be bound by any particular mechanism of action, ANRIL-TDO is supposed to induce eviction of ANRIL from its genomic regions, which it regulates the expression. In this case, ANRIL is still able to act on the 9p21 locus in a context of "normal situation" but not on the additional genes when overexpressed (pathological situation).
- ANRIL-TDO is a double-stranded DNA molecule and is therefore not sensitive to ribonucleases. It therefore theoretically has better pharmacokinetic properties than siRNAs which are in addition not optimal for targeting nuclear IncRNAs. Moreover, it is possible to synthesise it with the addition of chemical modifications to increase its biostability, its resistance to deoxynucleases contained in serum, its cellular adsorption or its nuclear localisation (Crinelli, R. et al. "Locked Nucleic Acids (LNA): Versatile Tools for Designing Oligonucleotide Decoys with High Stability and Affinity." Current Drug Targets 5, no. 8 (November 2004): 745-52 ([3])).
- ANRIL-TDO theoretically offers the advantage of not activating the innate immune system.
- ANRIL-TDO offers the advantage that it can theoretically be efficiently assimilated by the cells by liposomal vectorisation, an option currently favoured by the scientific community due to its proven efficacy (Hecker, Markus, and Andreas H. Wagner. "Transcription Factor Decoy Technology: A Therapeutic Update." Biochemical Pharmacology 144 (15 2017): 29-34 ([4])).

Accordingly, in a first aspect, the present invention provides an isolated double stranded DNA polynucleotide that forms triplex with sequence 5'-GGUGGCAGCAAGAGAAAAAUGAGGAAGAAGCAAAAGCGGAAA-3' (SEQ ID NO: 1) of the long non-coding RNA ANRIL (Antisense Non-coding RNA in the INK4 Locus).

As explained above, the sequence SEQ ID NO: 1 is a region within exon 8 (the full sequence of which being represented as SEQ ID NO: 15) of ANRIL (DBD-Ex8 for DNA Binding Domain-Exon8) identified by the Applicants, which is predicted to form triplexes with 422 gene regions. "ANRIL" refers to the human gene located within the CDKN2B-CDKN2A gene cluster at chromosome 9p21 (Gene ID: 100048912 on NCBI).

"Triplex" refers herein to DNA/DNA:IncRNA triple helix structures formed when a IncRNA ANRIL accommodates the major groove of the double stranded DNA by Hoogsteen or reverse Hoogsteen hydrogen bonds in either parallel or anti-parallel orientation. According to the invention, the double stranded DNA is designed to form specifically triplex with sequence SEQ ID NO: 1. Advantageously, the specificity of triplex formation is firstly based on sequence complementarity via Hoogsteen bonds. Thus, any sequence which does not offer possibilities to form such bonds is not supposed to form triplex: only the T-AT, C+-GC, A-AT and G-GC triplets can be formed. This allows to increase the stringency of triplex formation, since only few combinations are possible. Mismatches are tolerated in a range of 15%, meaning 6 mismatches out of 42-bp in the case of ANRIL-TDO. Secondly, the specificity of triplex formation is based on length of the DBD region. Indeed, Ex8-DBD is long to 42 nucleotides. Thus, longer is the ANRIL-TDO, lower is the probability to find an RNA region able to match as DNA binder via triplex formation. This ensures the stringency and the specificity of the triplex formation and therefore the specificity of the molecule.

According to the invention, "specifically" means that the double stranded DNA of the invention may not form triplex with any off-targets RNA, i.e. unintended target RNA sequences, in particular any other IncRNA than ANRIL. Specificity of the double stranded DNA may exist in spite of some mismatches with the sequence SEQ ID NO:1. In one embodiment, the triplex may contain no more than 15% mismatches, but forms triplex over at least about 70% of the length of the double stranded DNA polynucleotide. In another embodiment, the triplex is formed over at least about 80% of the length of the double stranded DNA polynucleotide, or over at least about 90%-95%, or over at least about 96%-98%. In certain embodiments, the double-stranded oligonucleotide of the invention contains at least or up to 1, 2, 3, 4, 5, 6, 7, 8, 9, 10, 11, 12, 13, 14, or 15 mismatches.

The isolated double stranded DNA polynucleotide of the invention may be a natural or artificial polynucleotide sequence. An artificial polynucleotide sequence may be produced by any means known by the skilled person, as chemical oligonucleotide synthesis or base pair synthesis, for example by the phosphoramidite method.

Advantageously, the isolated double stranded DNA polynucleotide of the invention may have a sense oligonucleotide having at least 85% sequence identity with sequence 5'-AAAGGCGAAAACGAAGAAGGAGTAAAAAGAGAACGACGGTGG-3' (SEQ ID NO: 2).

The percentage of identity may be of 85%, or 86%, 87%, 88%, 89%, 90%, 91%, 92%, 93%, 94%, 95%, 96%, 97%, 98%, 99% or 100%. For example, the sense oligonucleotide may consist of sequence 5'-AAAGG**G**GAAAA**G**GAAGAAGG**A**GAAAAAAGAGAA**G**GA**G**GG**A**GG-3' (SEQ ID NO: 4).

Advantageously, the isolated double stranded DNA polynucleotide of the invention may have an antisense oligonucleotide having at least 85% sequence identity with sequence 5'-CCACCGTCGTTCTCTTTTTACTCCTTCTTCGTTTTCGCCTTT-3' (SEQ ID NO: 3).

The percentage of identity may be of 85%, or 86%, 87%, 88%, 89%, 90%, 91%, 92%, 93%, 94%, 95%, 96%, 97%, 98%, 99% or 100%. For example, the antisense oligonucleotide may consist of sequence 5'-CCTCCCTCCTTCTCTTTTTTCTCCTTCTTCCTTTTCCCCTTT-3' (SEQ ID NO: 5).

For example, the isolated double stranded DNA polynucleotide of the invention may have:
- a sense oligonucleotide consisting of sequence 5'-AAAGGCGAAAACGAAGAAGGAGTAAAAAGAGAACGACGGTGG-3' (SEQ ID NO: 2), and
- an antisense oligonucleotide consisting of sequence 5'-CCACCGTCGTTCTCTTTTTACTCCTTCTTCGTTTTCGCCTTT -3' (SEQ ID NO: 3).

In another example, the isolated double stranded DNA polynucleotide of the invention may have:
- a sense oligonucleotide consisting of sequence 5'-AAAGGGGAAAAGGAAGAAGGAGAAAAAAGAGAAGGAGGGAGG-3' (SEQ ID NO: 4), and/or
- an antisense oligonucleotide consisting of sequence 5'-CCTCCCTCCTTCTCTTTTTTCTCCTTCTTCCTTTTCCCCTTT-3' (SEQ ID NO: 5).

The isolated double stranded DNA polynucleotide of the invention may comprise at least one modification allowing an enhancement of biostability. In some embodiments, at least 50% of the nucleotides in the isolated double stranded DNA polynucleotide are modified. For example, at least 50%, or at least 60%, or at least 70%, or at least 80%, or at least 90% of the nucleotides. In some embodiments, 100% of the nucleotides in the isolated double stranded DNA polynucleotide are modified. It may be biostability-enhancing chemical modifications such as locked nucleic acids, i.e. a modified nucleotide in which the ribose moiety is modified with an extra bridge connecting the 2' oxygen and 4' carbon and/or phosphorothioate bonds, i.e. bonds that substitute a sulfur atom for a non-bridging oxygen in the phosphate backbone of an oligo, for example between the last 3-5 nucleotides, at the 5'- or 3'-end of the oligo to inhibit exonuclease degradation. Advantageously, locked nucleic acids may be used according to Crinelli et al. ([3]).

The isolated double stranded DNA polynucleotide of the invention may be administered alone or in conjunction with a vector.

Another object of the invention relates to a vector comprising a double stranded DNA polynucleotide of the invention.

Such vectors are used to facilitate the cellular uptake or targeting of the double stranded DNA polynucleotide, and/or improve the oligonucleotide's pharmacokinetic or toxicologic properties. Any art recognized vectors for in vivo gene delivery may be use for this purpose. For example, the vector may be chosen among polymers such as poly (D,L-lactide co-glicolide) or chitosan, liposomes, gelatin, lipid based nanoparticles, viruses, such as adenoviruses, adeno-associated viruses or retroviruses, and antibodies. The vector may be, for example, adapted from NF-kB TFD ODN coated polysaccharide based nanoparticles used by Wardwell et al. ("Immunomodulation of cystic fibrosis epithelial cells via NF-κB decoy oligonucleotide-coated polysaccharide nanoparticles", J Biomed Mater Res A., 2015 May; 103(5):1622-31 ([18])), engineered nanomaterials delivering material used by Farahmand et al. ("Suppression of chronic inflammation with engineered nanomaterials delivering nuclear factor κB transcription factor decoy oligodeoxynucleotides", Drug. Deliv.. 2017 Nov;24(1):1249-1261 ([19])), intra-articular injection as in Sotobayashi et al. ("Therapeutic effect of intra-articular injection of ribbon-type decoy oligonucleotides for hypoxia inducible factor-1 on joint contracture in an immobilized knee animal model". J Gene Med, 2016 Aug;18(8):180-92 ([19])), or intrathecal administration described in Mamet et al. ("Pharmacology, pharmacokinetics, and metabolism of the DNA-decoy AYX1 for the prevention of acute and chronic post-surgical pain", Mol Pain. 2017 Jan;13:1744806917703112 ([20])).

Another aspect of the invention relates to a pharmaceutical composition comprising a double stranded DNA polynucleotide of the invention, or a vector as defined above.

The pharmaceutical composition may comprise pharmaceutically acceptable excipient that may include appropriate solvents, dispersion media, coatings, antibacterial and antifungal agents, isotonic and absorption delaying agents, and the like. The use of such media and agents for pharmaceutical active substances is well known in the art. Except insofar as any conventional media or agent is incompatible with the active ingredient, it can be used in the therapeutic compositions.

In some embodiments, the pharmaceutical composition may comprise at least one additional therapeutic agent. Non-limiting examples of additional therapeutic agents include but are not limited to nucleic acids (e.g., sd-rxRNA, etc.), small molecules (e.g. , small molecules useful for treating cancer, neurodegenerative diseases, infectious diseases, autoimmune diseases, etc.), peptides (e.g. , peptides useful for treating cancer, neurodegenerative diseases, infectious diseases, autoimmune diseases, etc.), and polypeptides (e.g., antibodies useful for treating cancer, neurodegenerative diseases, infectious diseases, autoimmune diseases, etc.). Compositions of the disclosure can have, in some embodiments, 2, 3, 4 or more additional therapeutic agents.

With respect to in vivo applications, the formulations of the present invention can be administered to a patient in a variety of forms adapted to the chosen route of administration, e.g. parenterally, orally, or intraperitoneally. Parenteral administration, may include administration by the following routes: intravenous; intramuscular; interstitial; intra-arterial; subcutaneous; intra-ocular; intrasynovial; trans-epithelial, including transdermal; pulmonary via inhalation; ophthalmic; sublingual and buccal; topically, including dermal; ocular; rectal; and nasal inhalation via insufflation.

The double stranded DNA polynucleotides, when it is desirable to deliver them systemically, may be formulated for parenteral administration by injection, e.g. by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Pharmaceutical preparations for parenteral administration may include aqueous solutions of the active compounds in water-soluble or water-dispersible form. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides. The oligonucleotides of the invention can be formulated in liquid solutions, preferably in physiologically compatible buffers such as Hank's solution or Ringer's solution. In addition, the oligonucleotides may be formulated in solid form and redissolved or suspended immediately prior to use. Lyophilized forms are also included in the invention.

Pharmaceutical preparations for topical administration include transdermal patches, ointments, lotions, creams, gels, drops, sprays, suppositories, liquids and powders. In addition, conventional pharmaceutical carriers, aqueous, powder or oily bases, or thickeners may be used in pharmaceutical preparations for topical administration.

Pharmaceutical preparations for oral administration include powders or granules, suspensions or solutions in water or non-aqueous media, capsules, sachets or tablets. In addition, thickeners, flavoring agents, diluents, emulsifiers, dispersing aids, or binders may be used in pharmaceutical preparations for oral administration.

For transmucosal or transdermal administration, penetrants appropriate to the barrier to be permeated are used in the formulation. Such penetrants are known in the art, and include, for example, for transmucosal administration bile salts and fusidic acid derivatives, and detergents. Transmucosal administration may be through nasal sprays or using suppositories. For oral administration, the oligonucleotides are formulated into conventional oral administration forms such as capsules, tablets, and tonics. For topical administration, the oligonucleotides of the invention are formulated into ointments, salves, gels, or creams as known in the art.

For administration by inhalation, such as by insufflation, the double stranded DNA polynucleotides according to the present invention may be delivered in the form of an aerosol spray presentation from pressurized packs or a nebulizer, with the use of a suitable propellant, e.g. dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

Also contemplated herein is pulmonary delivery of the double stranded DNA polynucleotides. The double stranded DNA polynucleotides may be delivered to the lungs of a mammal while inhaling and traverses across the lung epithelial lining to the blood stream.

Nasal delivery of a pharmaceutical composition of the present invention is also contemplated. Nasal delivery allows the passage of a pharmaceutical composition of the present invention to the blood stream directly after administering the therapeutic product to the nose, without the necessity for deposition of the product in the lung.

Other aspects of the invention relates to the use of sequence SEQ ID NO : 1 of ANRIL in a method of preparation of a double stranded DNA polynucleotide of the invention, and to a method of preparation of a double stranded DNA polynucleotide of the invention, comprising a step of synthesizing or isolating a double stranded DNA polynucleotide forming triplex with sequence SEQ ID NO : 1 of ANRIL.

Another object of the invention relates to an isolated double stranded DNA polynucleotide of the invention, for use in the treatment of myocardial infarction, aneurysms, stenosis, myocardial infarction, aneurysms, cancers, eye diseases or type 2 diabetes.

Advantageously, this treatment involves modulating IncRNA activity in a cell or a subject. The treatment comprises the step of contacting the cell or the subject with the isolated double stranded DNA polynucleotide of the invention in an amount effective to modulate IncRNA activity.

The isolated double stranded DNA polynucleotide of the invention may be administered to subjects or contacted with cells in a biologically compatible form suitable for pharmaceutical administration. By "biologically compatible form suitable for administration" is meant that the polynucleotide is administered in a form in which any toxic effects are outweighed by the therapeutic effects of the oligonucleotide. In one embodiment, oligonucleotides can be administered to subjects.

The useful dosage to be administered and the particular mode of administration will vary depending upon factors as the cell type, the age, weight and the particular subject and region thereof to be treated, the particular oligonucleotide and delivery method used, the therapeutic or diagnostic use contemplated, and the form of the formulation, for example, suspension, emulsion, micelle or liposome, as will be readily apparent to those skilled in the art. Typically, dosage is administered at lower levels and increased until the desired effect is achieved.

Examples of subjects include mammals, e.g. humans and other primates.

A method for treating a disease involving expression of IncRNA ANRIL, comprising modulating IncRNA ANRIL activity in a cell or a subject, by contacting the cell or the subject with the isolated double stranded DNA polynucleotide of the invention in an amount effective to modulate IncRNA ANRIL expression and/or activity.

This invention is further illustrated by the following examples with regard to the annexed drawings that should not be construed as limiting.

### Brief description of the figures

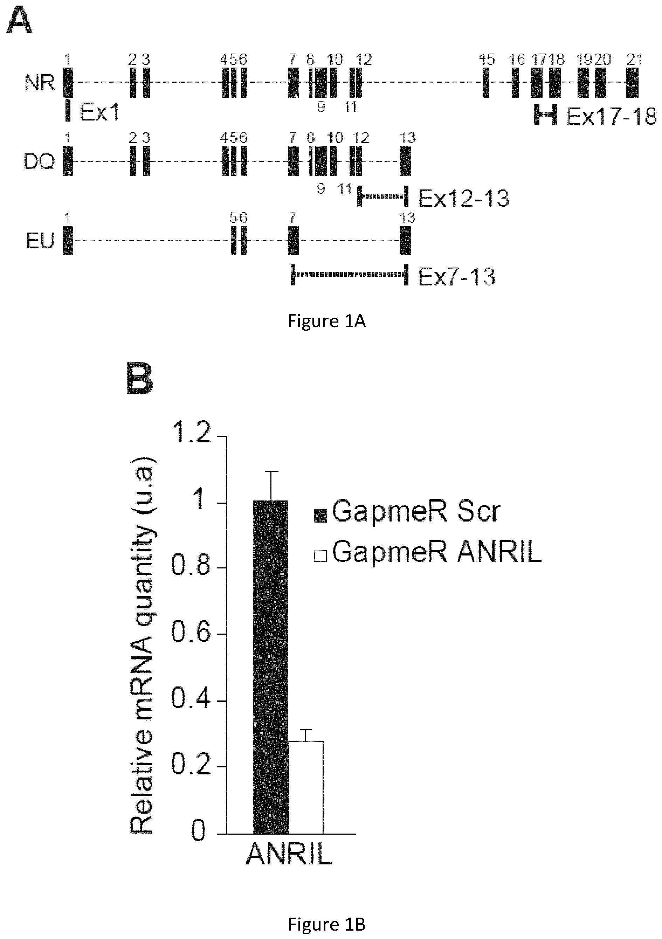

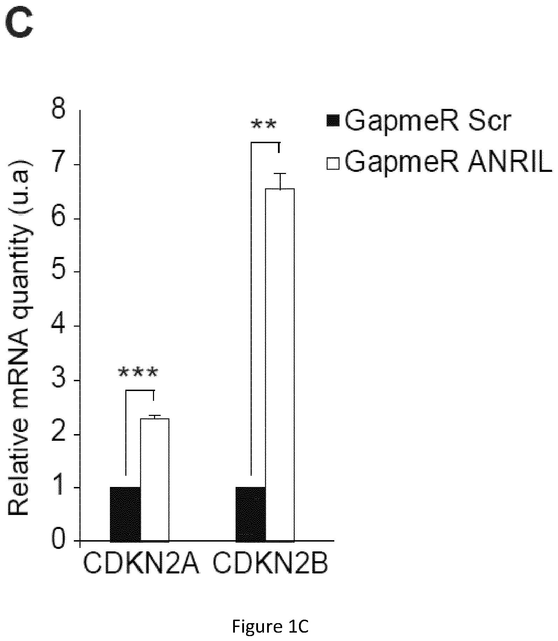

- Figure 1: represents (A) Schematic representation of the hybridization position of the 4 different LNA GapmeRs used to silence ANRIL. (B) RTqPCR analysis after LNA GapmeR transfection revealed up to 75% reduction in ANRIL's expression (n=3). Values are normalized to the GAPDH housekeeping gene. Relative RNA quantity in u.a.; GapmeR Scr (scrambled) in back; GapmeR ANRIL in white. (C) RTqPCR analysis of CDKN2A and CDKN2B expression following ANRIL knockdown by LNA GapmeRs (n=3). Values are normalized to the GAPDH housekeeping gene. Relative RNA quantity in u.a.; GapmeR Scr (scrambled) in back; GapmeR ANRIL in white.

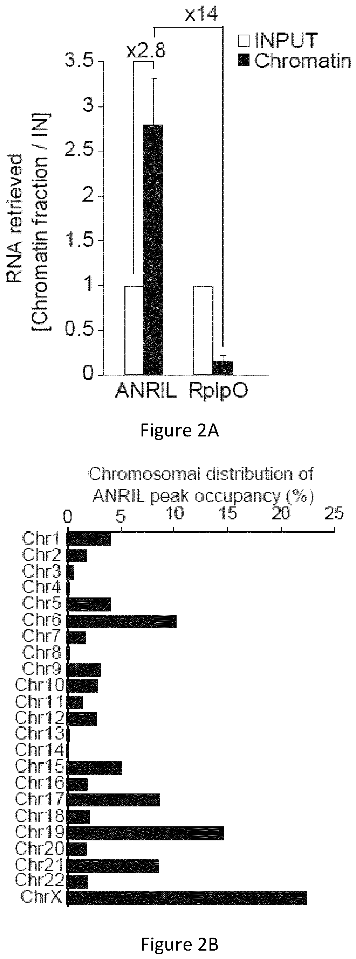

• Figure 2: represents (A) RNA extraction experiments showing ANRIL enrichment in the chromatin fraction (n=3). Values are normalized to Input (Input in white, Chromatin in black). RNA retrieved [Chromatin fraction/IN], (B) Chromosomal distribution of ANRIL peak occupancy (%), for each chromosome. ANRIL does not coat all the chromosomes at the same extent. Approximately 20% of the ANRIL peaks are localized on the X chromosome.

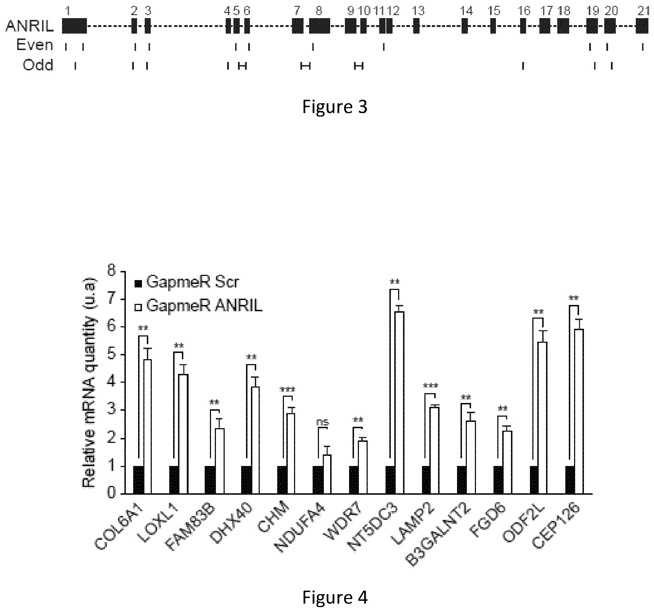

• Figure 3: represents the schematic representation of the biotinylated antisense oligos tiling ANRIL which are grouped into even and odd pools based on their position of hybridization. They have been used in the ChIRP-seq approach.
• Figure 4: represents RTqPCR validation of differentially expressed genes (COL6A1, LOXL1, FAM83B, DHX40, CHM, NDUFA4, WDR7, NT5DC3, LAMP2, B3GALNT2, FGD6, ODF2L and CEP126) upon ANRIL knockdown (n=5). Values are normalized to the GAPDH housekeeping gene. Relative RNA quantity in u.a.; GapmeR Scr (scrambled) in back; GapmeR ANRIL in white. Data are represented as mean ± SEM. P-values: moderated t-statistics, *p<0.05, **p<0.01, ***p<0.001, ns: not significant.

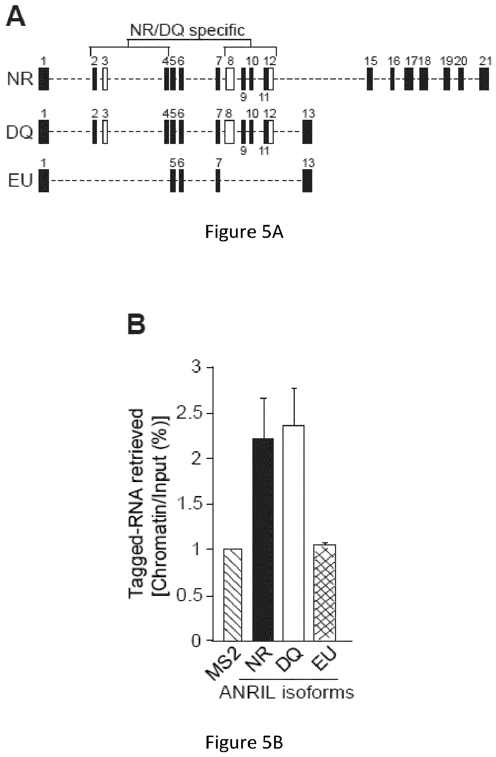

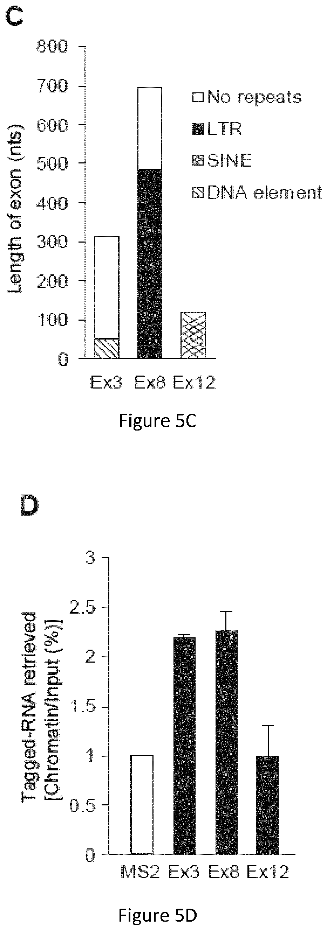

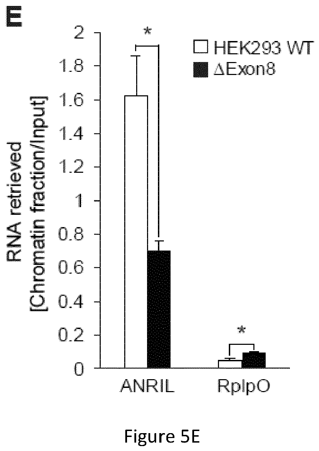

• Figure 5: represents (A) Schematic representation of the three major ANRIL isoforms NR, DQ, and EU. Exons and introns are represented by numbered white rectangles and black rectangles, respectively. (B) RNA extraction experiments after transient overexpression of the MS2 (Dashed lines)-tagged NR (black), DQ (white), and EU (lattices) isoforms (n=3). This identified the NR and DQ isoforms as DNA/chromatin binders but not the EU compared to the MS2-CTL. Values are normalized to Input (Tagged-RNA retrieved [Chromatin/Input (%)]. (C) RepeatMasker analysis showing the distribution of TEs in ANRIL's exons 3 (length: 313-nts), 8 (length: 696-nts), and 12 (length: 119-nts) (Length of exon in nts; No repeat in white, LTR in black, SINE in lattices, DNA element in dashed lines). (D) RNA extraction experiments after transient overexpression of the MS2-tagged exons 3, 8 and 12 (n=2). This identified the exons 3 and 8 of ANRIL as DNA/chromatin binders but not the exon 12 compared to the MS2-CTL. Values are normalized to Input (Tagged-RNA retrieved [Chromatin/Input (%)]. (E) RNA extraction experiments from ΔExon8 HEK293 cell lines (ΔExon8 cell lines in black, HEK293 WT in white) which revealed a reduction in chromatin association of ANRIL by 60% but not for RplpO compared to the HEK293 WT cell lines (n=3). Data are represented as mean ± SEM. P-values: moderated t-statistics, *p<0.05, **p<0.01, ***p<0.001 (%).

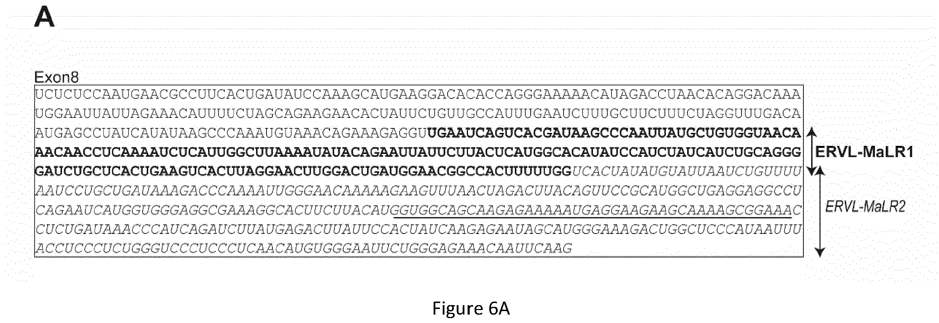

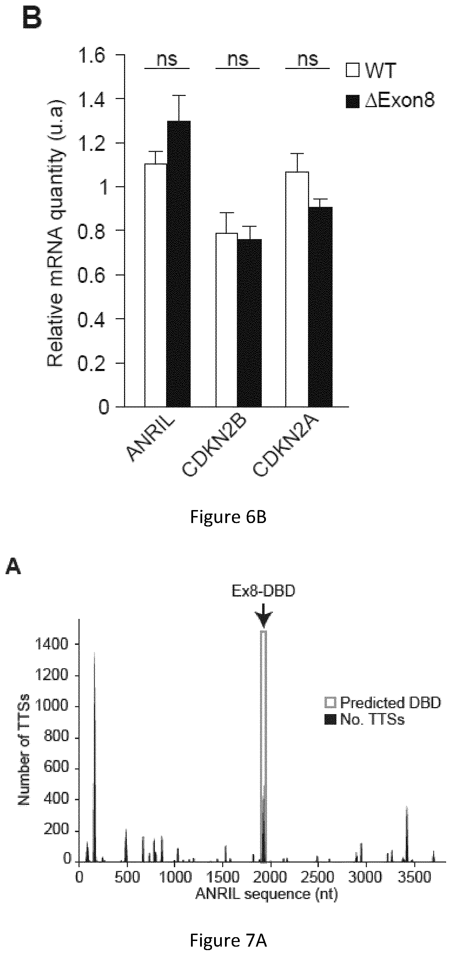

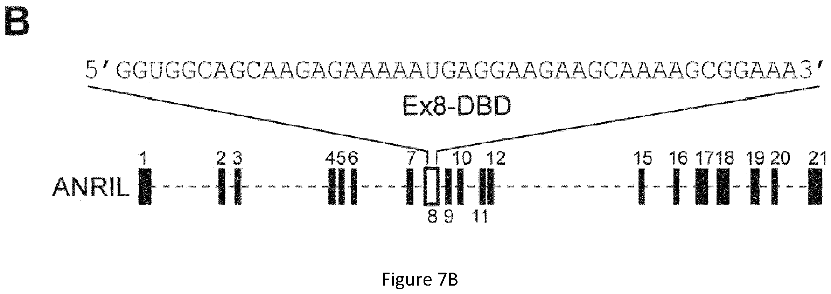

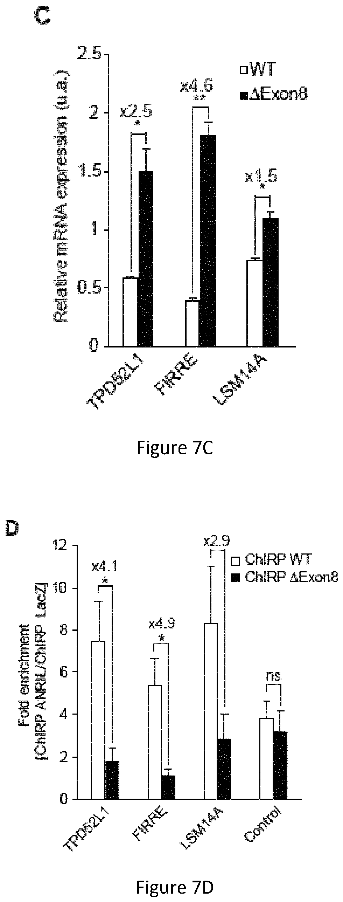

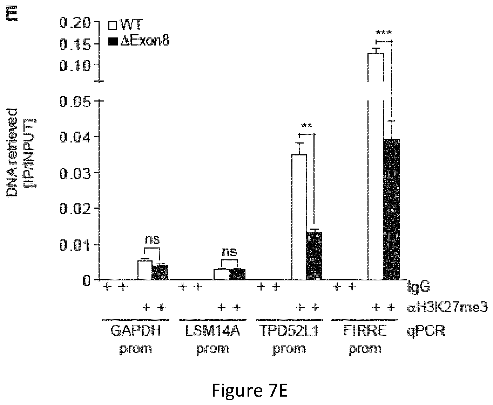

• Figure 6 represents (A) Exon8 full length sequence is 70% covered by two LTR/ERVL-MaLR elements highlighted in bold and underlined. The DBD identified by TDF analysis is present within the second LTR/ERVL-MaLR element and is highlighted in brown (related to Figure 7). (B) RTqPCR analysis of the mRNA levels of ANRIL, CDKN2B and CDKN2A in ΔExon8 HEK293 cells (n=4). Relative RNA quantity in u.a., ΔExon8 cell lines in black, HEK293 WT in white). No significant changes were observed upon deletion of the Exon8 on the expression of the tested genes. Data are represented as mean ± SEM.
• Figure 7 represents (A) TDF prediction using ANRIL full-length against the ChIRP-seq dataset (Number of TTSs as a function of ANRIL sequence (nt) for Predicted DBD in white and No. TTSs in black). This revealed the potential DBD on ANRIL's sequence located in Exon8 (p-value=0.0013) with its associated TTS (n=422) hereafter called ChIRP-seq TTSs. (B) Schematic representation of the position and purine-rich sequence of Ex8-DBD. (C) RTqPCR validation of differentially expressed genes in ΔExon8 HEK293 cells (n=5) (Relative RNA quantity in u.a., ΔExon8 cell lines in black, HEK293 WT in white). (D) ChIRP-qPCR on FIRRE, TPD52L1 and LSM14A loci shows that in the absence of Exon8, ANRIL dissociates from these loci (n=3). (Fold enrichment [ChIRP ANRIL/ChIRP LacZ], ΔExon8 cell lines in black, HEK293 WT in white). Values are normalized to the Input then fold enrichment is calculated by normalizing to LacZ. (E) ChIP-qPCR using H3K27me3 and control IgG antibodies on promoter regions of FIRRE, TPD52L1, and LSM14A (n=4). GAPDH used as a negative control. Values are normalized to the Input. (DNA retrieved [IP/INPUT], ΔExon8 cell lines in black, HEK293 WT in white). Data are represented as mean ± SEM. P-values: moderated t-statistics, *p<0.05, **p<0.01, ***p<0.001, ns: not significant.

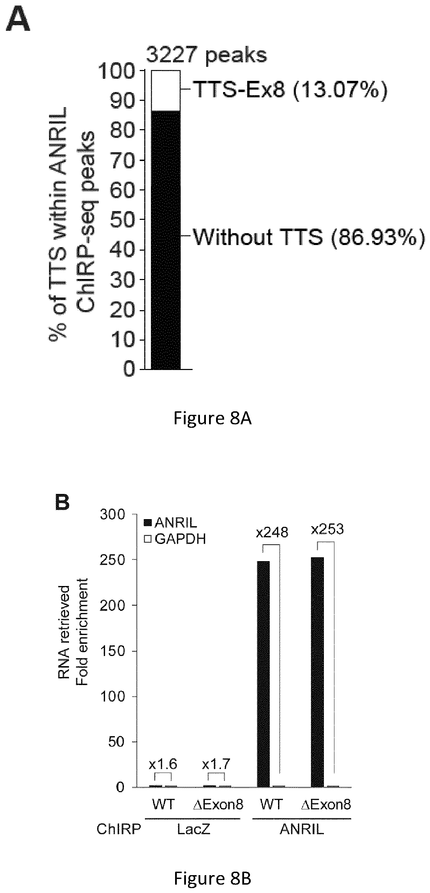

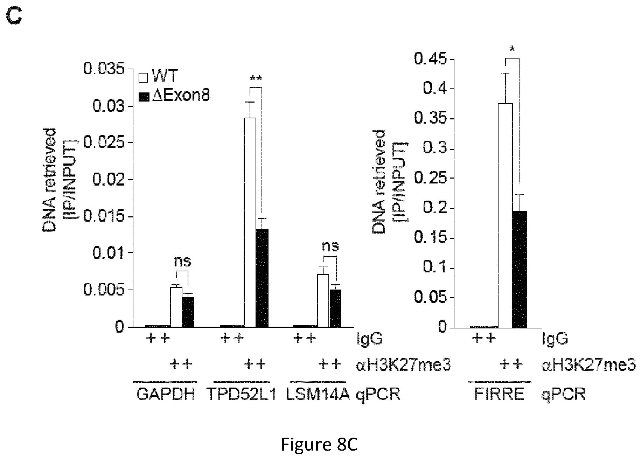

• Figure 8 represents (A) the percentage of TTS within ANRIL ChIRP-seq peaks: 13.07% of the 3227 ANRIL ChIRP-seq peaks contained predicted TTSs targeted by the DBD of Exon8. (B) Quality control showing the specific and efficient retrieval of ANRIL by using biotinylated probes in ΔExon8 ChIRP experiments. ANRIL (black) and the GAPDH (white) mRNA, used as negative control, from the Input and pulled-down fractions have been analyzed by RTqPCR. Values were normalized to Input. (C) ChIP-qPCR using H3K27me3 and IgG antibodies on the regions of FIRRE, TPD52L1, and LSM14A directly contacted by ANRIL (n=4) (DNA retrieved [IP/INPUT], ΔExon8 cell lines in black, HEK293 WT in white). GAPDH used as a negative control. Values are normalized to the Input. Data are represented as mean ± SEM. P-values: moderated t-statistics, *p<0.05, **p<0.01, ***p<0.001, ns: not significant.

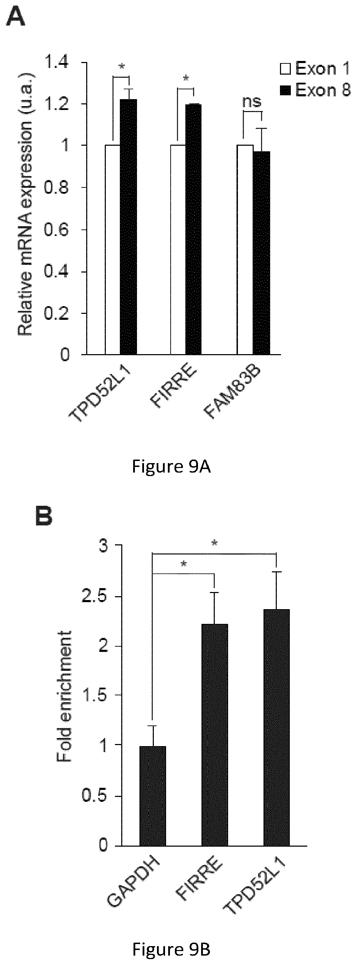

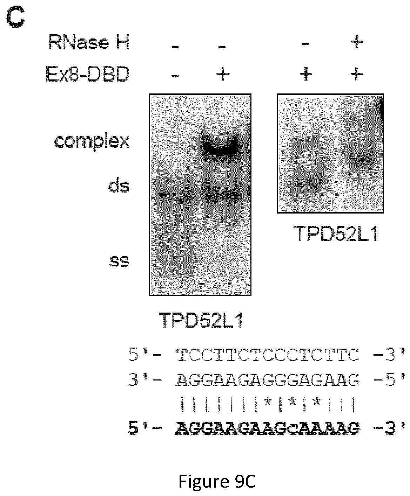

• Figure 9 represents (A) Relative expression (u.a.) of FIRRE, and TPD52L1 after Exon8 (black) and Exon1 (white) overexpression (n=3). FAM83B used as a negative control. Values are normalized to GAPDH housekeeping gene. (B) Fold enrichment in GAPDH, FIRRE and TPD52L1. (C) EMSA using 14 µM of synthetic Ex8-DBD (42 nts) with 100 fmol of double-stranded 32P-labeled double stranded oligonucleotides harboring a predicted TTS of TPD52L1. Gel shift was resistant to RNase H indicating a Hoogsteen base pairing. Potential Hoogsteen base pairing between Ex8-DBD represented for SEQ ID NO: 16 and TPD52L1 dsDNA sequences (SEQ ID NO: 17 and SEQ ID NO: 18) are shown; mismatches are marked *. Data are represented as mean ± SEM. P-values: moderated t-statistics, *p<0.05, **p<0.01, ***p<0.001, ns: not significant.

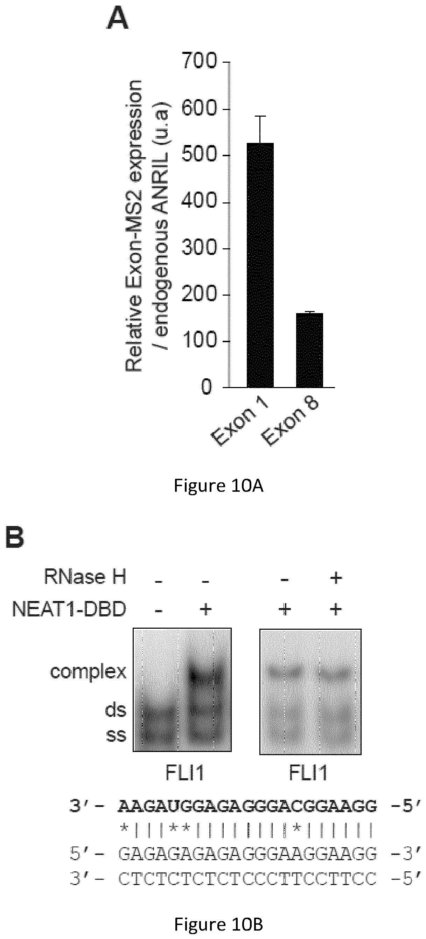

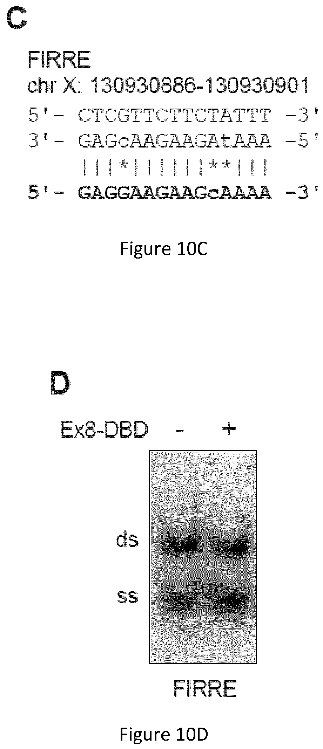

• Figure 10 represents (A) Relative expression (u.a.) of Exon1 and Exon8 following their transient overexpression in HEK293 cells (n=3). Values are normalized to GAPDH housekeeping gene. Data are represented as mean ± SEM. (B) EMSA using 14 µM of synthetic NEAT1-DBD (40 nts) with 100 fmol of double-stranded 32P-labeled oligonucleotides harboring a TTS of FLI1. Gel shift was resistant to RNase H indicating a Hoogsteen base pairing. Potential Hoogsteen base pairing between NEAT1-DBD represented for SEQ ID NO: 19 and FLI1 dsDNA sequences (SEQ ID NO: 20 and SEQ ID NO: 21) are shown; mismatches are marked (*). (C) Putative Hoogsteen base pairing between Ex8-DBD represented for SEQ ID NO: 22 and the predicted FIRRE dsDNA sequences (SEQ ID NO: 23 and SEQ ID NO: 24) are shown; mismatches are marked (*). (D) EMSA using 14 µM of synthetic Ex8-DBD with 100 fmol of double-stranded 32P-labeled oligonucleotides harboring a TTS of FIRRE.

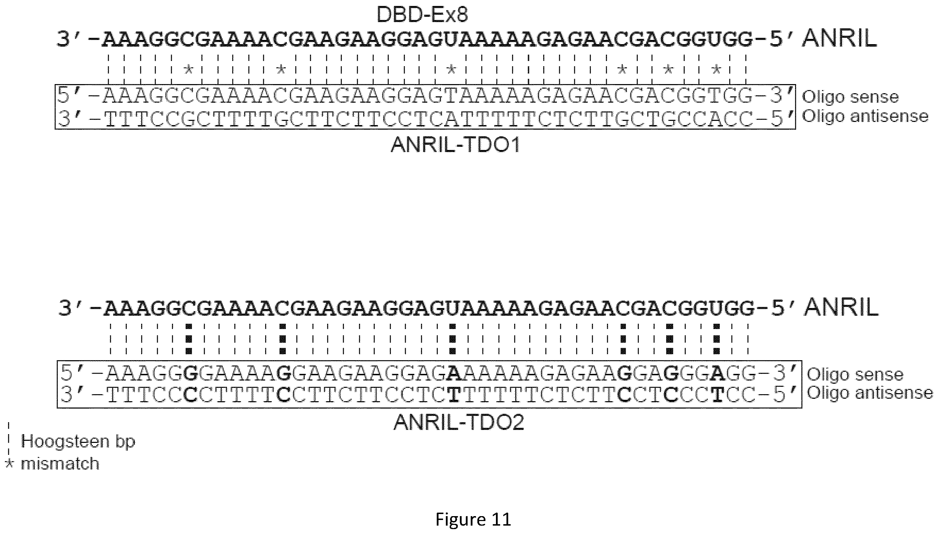

• Figure 11 represents the Hoogsteen base pairs, and misappariements, between DBD-Exon8 (SEQ ID NO : 1) and ANRIL-TDO1 (having a sense oligonucleotide consisting of sequence SEQ ID NO: 2 and an antisense oligonucleotide consisting of sequence SEQ ID NO: 3) and ANRIL-TDO2 (a sense oligonucleotide consisting of sequence SEQ ID NO: 4 and an antisense oligonucleotide consisting of sequence SEQ ID NO: 5).

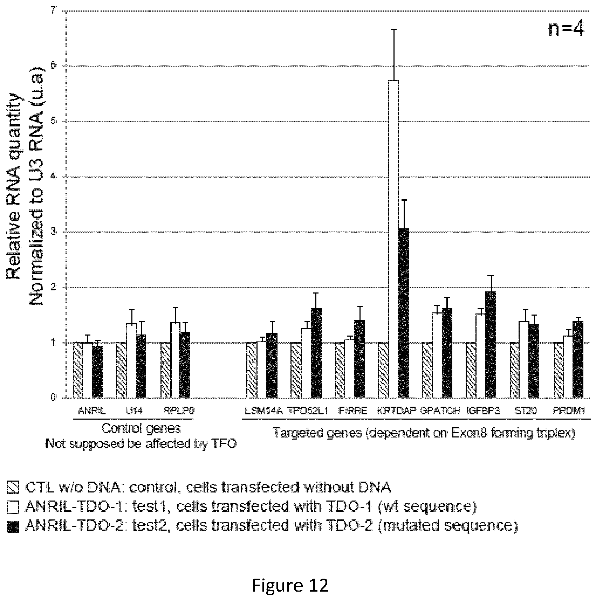

• Figure 12 shows RNA quantity (a.u.) normalized to U3, for CTL w/o DNA (Dashed), ANRIL-TDO1 (white) and ANRIL-TDO2 (black) (n=4), for genes LSM14A, TPD52LA, FIRRE, KRTDAP, GPATCH, IGFBP3, ST20 and PRDM1. ANRIL, U14 and RPLPO were used as controls.

### Examples

### Example 1: Exon8 of ANRIL largely contributes to ANRIL genomic association and to the trans-regulation of 9 of the 123 primary genes

Transposable elements (TEs) are the major contributors to the bulk of the genomic DNA in mammals. They can provide novel regulatory sequences such as promoters and enhancers. Recently, several studies focused on the possible relationship between TEs and IncRNA functions. This revealed that nearly half of the IncRNA sequences (41%) are derived from TEs. Interestingly, IncRNA exons are strongly and non-randomly enriched in Endogenous RetroViruses (ERVLs) belonging to the LTR class, while other classes of TEs, like SINE (Alu) and LINE (LINE1 and LINE2) are under-represented. It was shown for several IncRNAs that the presence of TEs termed RIDLs (Repeat Insertion Domains of Long noncoding RNAs) impacts their localization and/or functions. Furthermore, Holdt and coll. identified Alu sequences within ANRIL and within 5 kb regions of gene promoters affected by the overexpression of ANRIL sub-fragments, suggesting that TEs within ANRIL sequence might be involved in its trans-regulatory activities.

In the present study, we investigated whether TEs participate in ANRIL's chromatin recognition necessary for gene *trans*-silencing. We identified genome-wide the chromatin occupancy of ANRIL in HEK293 cells by applying the ChIRP-seq approach and found that ANRIL associates with 3227 binding sites mostly composed by G/A residues. By crossing the ChIRP-seq with transcriptomic data from ANRIL knocked-down cells, we established a list of 188 genes corresponding to primary *trans*-targets of ANRIL, since they were both contacted by ANRIL and affected in terms of expression. Among them, 123 genes were found to be negatively regulated by ANRIL possibly through its PcG-mediated *trans-*regulatory activity. *In silico* approaches highlighted the presence of multiple classes of TEs throughout ANRIL exons. In particular, 70% of the longest Exon8 was made up of ERVL elements. We investigated its putative role in ANRIL's *trans*-activity. We showed that its presence is required for the association of ANRIL to the chromatin, since Exon8 deletion resulted in a severe reduction of ANRIL's genomic occupancy. By applying highly stringent criteria, we accurately identified 9 out of the 123 *trans*-target genes of ANRIL, which expression specifically depends on the presence of Exon8. By further *in silico, in cellulo* and *in vitro* characterization, we showed that Exon8 contains a 42-nts sequence, which is likely to contribute to both recognition and silencing of the FIRRE and TPD52L1 genes. We brought evidences in favor of a recognition mode involving direct DNA/DNA:RNA complex formation. Overall, our data showed that ANRIL contains ERVL-enriched domain in Exon8 involved in its specific chromatin targeting. This reinforces the emergent role of TEs in processes engaged by nuclear IncRNAs to recognize the chromatin in a specific manner.

### MATERIALS AND METHODS

### Cell culture

Human Embryonic Kidney (HEK293) cells were grown in Dulbecco's Modified Eagle's Medium-high glucose (DMEM) (Sigma-Aldrich) supplemented with 10% Fetal Bovine Serum (FBS) (Sigma-Aldrich), 1% penicillin/streptomycin (Sigma-Aldrich), and 1% L-glutamine (Sigma-Aldrich).

### Generation of knocked-out cells by CRISPR/Cas9 approach

Two sgRNAs targeting the 5' and 3' extremities of Exon8 were designed using the CHOPCHOP website (https://chopchop.cbu.uib.no/) and inserted into the pSpCas9BB-2A-puro (Ran,F.A. et al.: "Genome engineering using the CRISPR-Cas9 system". Nat. Protoc., 8 (2013), 2281-2308 ([5])). The two vectors containing the sgRNAs were co-transfected into the HEK293 cells using lipofectamine 2000 (Invitrogen) according to the manufacturer's recommendations. Clonal selections were performed according to the manufacturer's recommendations. Clones were then isolated and DNA was extracted followed by end point PCR screening for homozygous deletions. Positive clones were verified by sequencing. The oligonucleotides used for deletion of Exon8 by CRISPR-Cas9 are listed in Table 1

**Table 1 :**

| Primer Name | Position | Sequence (5'-3') |
|---|---|---|
| sgRNA Exon8 5' | Fw | CACCGATATCAGTGAAGGCGTTCAT (SEQ ID NO : 6) |
| sgRNA Exon8 5' | Rv | AAACATGAACGCCTTCACTGATATC (SEQ ID NO : 7) |
| sgRNA Exon8 3' | Fw | CACCGACCCAGAGGGAGGTAAATTA (SEQ ID NO : 8) |
| sgRNA Exon8 3' | Rv | AAACTAATTTACCTCCCTCTGGGTC (SEQ ID NO : 9) |

### LNA GapmeRs transfection

LNA GapmeRs either targeting unique regions of ANRIL isoforms (Figure 1A) or non-targeting any region (scrambled, used as a negative control) were designed by QIAGEN. 500,000 HEK293 cells were seeded per well in 6 well-plates 12-16h before transfection. Transfection was performed using Lipofectamine 2000 (Invitrogen). A mix of the 4 ANRIL LNA GapmeRs or scrambled LNA GapmeRs was used for transfection at a final concentration of 25nM. All samples were collected 48h post-transfection in RLT lysis buffer (RNeasy mini kit QIAGEN) for total RNA extraction. The LNA GapmeR sequences are listed below:
GapmeR Scrambled: GCTCCCTTCAATCCAA (SEQ ID NO : 10)
GapmeR Exon1: TCAGAGGCGTGCAGCG (SEQ ID NO : 11)
GapmeR Exon17-18: TAAGATCCAGTGGTGG (SEQ ID NO : 12)
GapmeR Exon12-13: CGTAATCATCCATGCA (SEQ ID NO : 13)
GapmeR Exon7-13: AATCATCCTGTCAAA (SEQ ID NO : 14)

### Total RNA extraction and RTqPCR

Total RNAs were collected using RNeasy mini kit (QIAGEN) and extracted following the manufacturer's recommendation. Quantification of the extracted RNAs was done using the nanodrop 2000. DNase step was performed on 1.25 µg of RNA for 1h at 37°C using DNase I recombinant, RNase-free (Sigma-Aldrich). Then RNAs were reverse transcribed using the Superscript III kit (Thermo Fisher Scientific) following the manufacturer's recommendation. cDNAs were diluted 2.5 times in water and mRNA expression level was assessed by real time quantitative PCR (RTqPCR) using the iTaq^{™} Universal SYBR^{®} Green Supermix (Bio-Rad) and ViiA-7 Real-Time PCR system (Applied Biosystems). Transcript RNA levels were normalized against GAPDH reference gene following the relative standard curve method. The RTqPCR primers were used at 1 µM final concentration. The RTqPCR primers used in this study are listed in the Table 2.

### Microarray expression profiling

The integrity of the RNA was first validated by pico-chip bioanalyzer 2100 (EPI-RNA seq platform from IBSLor, UMS2008, France). Then 5 ng of RNA samples were analyzed using the Clariom D Human Assay Microarrays (Applied Biosystems) which includes transcriptome wide gene-and exon-level expression probesets. Microarray hybridization and scanning was conducted in IMoPA, France according to the manufacturer's standard protocols. Briefly, each purified RNA sample was transcribed to double-strand cDNA, followed by cRNA synthesis and biotin-labeling. The labeled cRNAs were then hybridized onto the Clariom D microarray. After washing, the arrays were scanned using the GeneChip Scanner 3000 (Applied Biosystems). Data analysis was performed using the Transcriptome Analysis Console (TAC). The signal obtained was normalized using the SST-RMA method and the annotation of the probe sets was done using the "Clariom_D_Human.r1.na36.hg38.a1.transcript.csv" annotation file obtained from Affymetrix. Differential expression was calculated using the "Limma" package (takes into consideration the low sample numbers) and the p-value was adjusted using the eBayes correction. Differentially expressed RNAs between condition and control were identified based on fold change and FDR.

### Chromatin preparation

5 millions of HEK293 cells were crosslinked in 1% methanol free formaldehyde (Thermo Fisher Scientific) for 10 min and then quenched with 0.125 mM glycine for 5 min. Samples were then lysed using the ChIRP lysis buffer (50 mM Tris-HCI pH 7.0, 10 mM EDTA, 1% SDS) supplemented with protease inhibitor cocktail 100x (Thermo Fisher Scientific) and Ribolock RNase inhibitor (Thermo Fisher Scientific). Samples were then sonicated using the Covaris M220 ultrasonicator and 25 µg of sheared chromatin was treated with 200 µg of proteinase K for 45 min at 50°C. DNA was then extracted using GeneJET Gel Extraction kit (Thermo Fisher Scientific) and quantified by the nanodrop 2000. 600 ng of the subsequent DNA were loaded on agarose gel 1.2% to verify the shearing efficiency. The sheared chromatin was then flash frozen in liquid nitrogen and stored at -80°C for later use.

### RNA extraction from chromatin

25 µg of sheared chromatin was treated with 200 µg of proteinase K for 45 min at 50°C. RNA was extracted from the treated chromatin using the RNeasy MinElute Cleanup kit (QIAGEN) according to the manufacturer's recommendation. DNase and reverse transcription were then performed as described above. cDNAs were diluted 10 times in water and ANRIL enrichment level was assessed by RTqPCR using the iTaq^{™} Universal SYBR^{®} Green Supermix (Bio-Rad) and ViiA-7 Real-Time PCR system (Applied Biosystems). Transcripts RNA levels were normalized against the Input.

### ChIRP-seq and data analysis

ChIRP antisense biotinylated probes were designed using online designer at www.singlemoleculefish.com against the ANRIL full-length sequence. 23 probes were generated tiling the whole IncRNA ANRIL and split into two independent even and odd probe pools based on their relative positions along ANRIL sequence. Similarly, 20 probes against LacZ mRNA were used as negative control. The ChIRP-seq probes used in this study are listed in the Supplementary Table S5. ChIRP-seq was performed on 30 µg of sheared chromatin followed by RNA elution using the RNeasy MinElute Cleanup kit (QIAGEN) and DNA elution using GeneJET Gel Extraction kit (Thermo Fisher Scientific) on two independent replicates. High-throughput sequencing libraries were constructed using the NEBNext Ultra II DNA Kit according to the manufacturer's recommendation (IBSLor Epitranscriptomics and Sequencing Core Facility, Nancy, France). Paired-end sequencing was done on the NextSeq 500 with a read length of 43 bp and with 45 million reads per sample (I2BC sequencing platform, Paris, France). Data analysis was adapted from the ChIRP-seq pipeline (Chu,C. et al. (2011) Genomic maps of lincRNA occupancy reveal principles of RNA-chromatin interactions. Mol. Cell, 44, 667-678 ([6])). Briefly, the fastq files of replicates 1 and 2 were aligned to the hg19 genome using bowtie2 (Langmead,B. and Salzberg,S.L. (2012) Fast gapped-read alignment with Bowtie 2. Nat. Methods, 9, 357-359 ([7])). Then the aligned reads of both even and odd bam files of each replicate were intersected and merged using bedtools (Quinlan,A.R. and Hall,I.M. (2010) BEDTools: a flexible suite of utilities for comparing genomic features. Bioinformatics, 26, 841-842 ([8])). Peak calling was then performed against LacZ negative control using MACS2 peak caller (Zhang,Y. et al. (2008) Model-based Analysis of ChIP-Seq (MACS). Genome Biol., 9, R137 ([9])). Peaks were further filtered based on the score ≥ 15, and FDR ≤ 0.05. Peaks located in blacklisted regions of the genome identified by ENCODE were discarded. Finally, only common peaks between both replicates were kept and considered as "True Peaks". The true peaks were annotated using the ChIPseeker package in R (Yu,G. et al. (2015) ChIPseeker: an R/Bioconductor package for ChIP peak annotation, comparison and visualization. Bioinformatics, 31, 2382-2383 (([10]). Peak distribution was calculated by normalizing the total length of peaks per chromosome by the size of their respective chromosome. Validation of several peaks was performed by quantitative PCR (qPCR) using the ViiA-7 Real-Time PCR system (Applied Biosystems). The qPCR primers were used at 1 µM final concentration.

### Chromatin Immunoprecipitation

ChIP experiments were performed in HEK293 cells according to the X-ChIP abcam protocol. Briefly, approximately 25 µg of sheared DNA was used per IP and incubated overnight with 3 µg of H3K27me3 antibody (Invitrogen) / Magna ChIP^{™} Protein A+G Magnetic Beads (Merck Millipore) complexes. The following day, the beads were subsequently washed in low salt wash (0.1% SDS, 1% Triton X-100, 2 mM EDTA, 20 mM Tris-HCl pH 8.0, 150 mM NaCl), high salt wash buffer (0.1% SDS, 1% Triton X-100, 2 mM EDTA, 20 mM Tris-HCl pH 8.0, 500 mM NaCl), and LiCl wash buffer (0.25 M LiCl, 1% NP-40, 1% Sodium Deoxycholate, 1 mM EDTA, 10 mM Tris-HCI pH 8.0). Samples were then treated with 200 µg of proteinase K in a total volume of 200 µL for 45 min at 50°C. DNA was prepared using the GeneJET Gel Extraction kit (Thermo Fisher Scientific) according to the manufacturer's recommendations, eluted in 15 µL of elution buffer and diluted 2 times with water. Primer list used can be found in Table 3.

### Motif analysis

The MEME package from MEME Suite was used to identify consensus DNA motifs enriched in the ANRIL ChIRP-seq peaks identified above (Bailey,T.L. and Elkan,C. (1994) Fitting a Mixture Model By Expectation Maximization To Discover Motifs In Biopolymer. Proc. Int. Conf. Intell. Syst. Mol. Biol., 2, 28-36 ([11]); Bailey,T.L. et al. (2009) MEME SUITE: tools for motif discovery and searching. Nucleic Acids Res., 37, W202-W208 ([12])). Default parameters were used as such:
1/ The width of the expected motif was set between 6 and 50.
2/ The expected occurrence per sequence was set to zero or one (zoops).
3/ The maximum number of motifs to search for was 5.

### Triple helix identification

Triplex Domain Finder (TDF) analysis was performed according to (Kuo,C.-C. et al. (2019) Detection of RNA-DNA binding sites in long noncoding RNAs. Nucleic Acids Res., 47, e32-e32 ([13])). Full length ANRIL sequence (FASTA format) and ChIRP-seq peaks (BED format) were used as inputs in the analysis. The genome used was the hg19 and the minimum length of triplex was set to 15.

### Electrophoretic mobility shift assays

Gel shift assays were performed as previously described (Sentürk Cetin et al. (2019) Isolation and genome-wide characterization of cellular DNA:RNA triplex structures. Nucleic Acids Res., 47, 2306-2321 ([14])). Briefly, purine rich strand DNA oligos were 5'-labeled with γ[³²P]ATP (Perkin Elmer) and annealed in equimolar ratios to their complementary pyrimidine rich strand DNA oligos in an annealing buffer 1x (10mM Tris-Acetate, 50 mM NaCl, 5 mM Mg-Acetate) for 2 min at 95°C and slowly cooled down to 20°C. For triplex formation, RNA was incubated with 100 fmol of radiolabeled duplex oligos for 1 h at 37°C in Triplex-buffer A (40 mM Tris-Acetate pH 7.4, 30 mM NaCl, 20 mM KCl, 5 mM Mg-Acetate, 10% glycerol, protease inhibitor cocktail 1x (Thermo Fisher Scientific), 20 U of Ribolock (Thermo Fisher Scientific)) in a final volume of 10 µL. Triplex formation was monitored by electrophoresis on 12% native polyacrylamide gels at 15 mA and revealed using a typhoon scanner.

### Transient transfection of ANRIL exons and isoforms

Calcium phosphate mediated transfection was used to overexpress separately ANRIL isoforms (NR, DQ, and EU) and exons 1, 3, 8, and 12 in the HEK293 cells according to the manufacturer's recommendations. Briefly, 360 000 HEK293 cells were seeded per well in 6 well-plates 12-16h before transfection. 1.5 µg of pcDNA3.1 expression vectors were used for transfection in 2 mL final volume. Samples were collected 48h post-transfection in RLT lysis buffer (RNeasy mini kit QIAGEN) for total RNA extraction.

### Triplex capture assay

This protocol was adapted from (Sentürk Cetin et al., ([14])). Briefly, RNA-free genomic DNA was sheared with Covaris M220 ultrasonicator to an average size of 200-500 bp and 75 µg of fragmented DNA were incubated with 40 pmol of in vitro transcribed Exon8 for 1 h at 30°C in 40 µL of Triplex buffer (10 mM Tris-HCl pH 7.4, 50 mM KCI, 5 mM MgCl₂) for triplex formation. The formed DNA-RNA complexes were incubated with 100 pmol of biotinylated probe complementary to Exon8 for 4hrs at 30°C and isolated using the MyOne Streptavidin C1 Dynabeads (Thermo Fisher Scientific). After 3 washes with 700 µL of wash buffer (10 mM Tris-HCl pH 7.4, 50 mM KCl, 5 mM MgCl₂, 0.05% Tween-20) DNA was eluted by incubation of the beads with 100 µL of elution buffer (150 mM NaCl, 12.5 mM EDTA, 100 mM Tris-HCl pH 7,5, 1% SDS) for 5 min at 75°C. DNA was then purified and concentrated using the GeneJET Gel Extraction kit (Thermo Fisher Scientific) according to the manufacturer's recommendations, eluted in 10 µL of elution buffer and diluted 2 times with water.

### RESULTS

### ANRIL Binds 3,227 Loci Across the Genome of HEK293 cells

We first evaluated the ability of ANRIL to associate with the chromatin fraction in HEK293 cells. Chromatin was prepared by formaldehyde cross-linking followed by shearing. RNAs associated with cross-linked chromatin and cellular RNAs (INPUT) were extracted and analyzed by RTqPCR (Sentürk Cetin et al., ([14])). We observed a relative enrichment of ANRIL in the chromatin fraction compared to the INPUT (2.8x) and to the unrelated RplpO transcript encoding a ribosomal protein (14x) (Figure 2A). We then assessed the genome-wide occupancy of ANRIL at high resolution by applying the ChIRP-seq approach ((Chu,C. et al., ([6]); Engreitz,J.M. et al. (2013) The Xist IncRNA exploits three-dimensional genome architecture to spread across the X-chromosome. Science, 341, 1237973 ([15])). By the use of tiling biotinylated antisense 20-mer oligos, we efficiently captured the endogenous ANRIL from chromatin (Figure 3).

When compared to the unrelated GAPDH mRNA and the negative control LacZ mRNA, which is not expressed in eukaryotic cells, ANRIL enrichments of 532- and 375-fold were observed for the even and odd probe pools, respectively. The purified DNA was then analyzed by high-throughput sequencing. Data analysis was done from 2 independent experiments as previously described (Chu,C et al. ([6])), followed by peak calling using MACS2 peak caller (Jeon,Y. and Lee,J.T. (2011) YY1 tethers Xist RNA to the inactive X nucleation center. Cell, 146, 119-133 ([16])). This allowed us to identify 3,227 ANRIL-peaks corresponding to the genomic sites for ANRIL occupancy. We built a representative ANRIL-peak (score ≥ 15, and FDR ≤ 0.05) found on the X chromosome that we validated by ChIRP-qPCR. Similar experiments validated the *9p21* locus used as positive control of ANRIL binder in addition to MX1 and STAT1 peaks we identified by ANRIL ChIRP-seq. No enrichment was observed for the TERC locus used as a negative control. Peak distribution analysis showed that almost all the chromosomes were contacted by ANRIL. Few peaks belonged to chromosomes 4, 8, 13, 14 and Y, while 15% (176) and 23% (754) of them were on the chromosomes 19 and X, respectively (Figure 2B). Additionally, most of the ANRIL-peaks were located in intergenic and intronic regions (1,608 and 1,301 respectively) compared to UTR, promoter or exonic regions. Based on human genome composition, no significant peak-enrichment was observed for any particular DNA subcategories. These results indicate that ANRIL is a nuclear IncRNA able to contact several loci dispersed throughout the genome of HEK293 cells.

To further characterize the interaction between ANRIL and the genome, motif analysis was performed on the 3,227 ANRIL ChIRP-seq peaks, using the MEME suite (http://meme-suite.org/). The most significant motif (E-value= 1.8e-048) corresponded to a highly predominant 21-bp long element present in 3,167 out of the 3,227 ANRIL ChIRP-seq peaks. Interestingly, this motif, mainly composed of G and A residues, shows a high degree of similarity with those previously identified by ChIRP-seq experiments as genomic binding sites for the IncRNAs roXes and HOTAIR (Chu,C et al. ([6])). We also looked for Alu motifs that were previously shown to be enriched within 5 kb fragments from promoter of multiple genes up- or down-regulated upon ANRIL overexpression. Interestingly, a similar Alu sequence was identified in motif 2 (41-bp long), that we detected for 48 genomic binding sites of ANRIL. Overall, our data suggest that purine-rich DNA regions and some TEs may be used as anchors by ANRIL for the recognition of specific genomic regions.

### In HEK293 Cells, ANRIL is Likely to Silence the Expression of 123 Genes in a Direct Manner

To characterize in depth ANRIL's *trans*-activity and to identify the genes directly regulated by ANRIL, we silenced the expression of the main ANRIL isoforms in HEK293 cells followed by genome-wide expression analysis. This was achieved by using a mix of 4 LNA GapmeRs (single stranded antisense oligos (ASO)) hybridizing to unique regions of the main ANRIL isoforms as such: GapmeR Exon1 (all isoforms), Exon17-18 (NR isoform), Exon12-13 (DQ isoform) and Exon7-13 (EU isoform) (Figure 1A). A reduction of 75% of ANRIL's level upon treatment of HEK293 cells with this GapmeR mix was achieved as compared to treatment with scrambled GapmeR (Figure 1B). This reduction was accompanied by a 2.2- and 6.5-fold increase of CDKN2A and CDKN2B mRNA levels, respectively (Figure 1C). These results were consistent with the ANRIL's cis-activity previously described (Kotake,Y. et al. (2011) Long non-coding RNA ANRIL is required for the PRC2 recruitment to and silencing of p15INK4B tumor suppressor gene. Oncogene, 30, 1956-1962 ([17]). Then, total RNAs were extracted and analyzed by next generation Clariom D microarrays from Affymetrix. Upon ANRIL knockdown, 2618 genes (1474 upregulated and 1144 downregulated with an FDR <0.01, log2FC>|1|) experienced changes in mRNA level. The effects observed on some of the genes upon ANRIL knockdown were further validated by RTqPCR (Figure 4).

Since it was documented that ANRIL associates with the PcG to silence genes, we postulated that a significant number out of the 1474 upregulated genes upon ANRIL's KD might be silenced by ANRIL through a similar repressive mechanism. Nevertheless, among genes with a modified level of expression, we had to identify which ones were the primary targets, because one primary target can regulate the expression of many downstream genes. We hypothesized that the genes being both affected and in direct contact with ANRIL in the chromatin structure are likely to be primary targets of ANRIL. We therefore compared the list of the 1474 upregulated genes with the ANRIL ChIRP-seq data and identified 123 genes filling conditions to be directly regulated (*p*<1.383e-12). Gene ontology analysis did not reveal any enriched pathways. We named these genes ANRIL direct *trans*-targets since they were both contacted and silenced by ANRIL and are consequently well suited to be regulated by ANRIL in a direct manner.

### TEs in Exon8 are Critical for ANRIL's Binding to the Genome and Gene Regulation of 9 ANRIL Direct Trans-Targets

Since the three major ANRIL isoforms are composed of different combinations of exons and are proposed to differentially affect gene expression, we postulated that each of them might contain unique functional domains (Figure 5A). In order to reveal the one(s) responsible for the binding of ANRIL to the genome, we first evaluated the ability of the NR, DQ and EU isoforms to associate with the chromatin fraction. RNA extraction from chromatin was performed after individual overexpression of these MS2-tagged isoforms by transient transfection in HEK293 cells (Figure 5B). This identified both NR and DQ isoforms as DNA/Chromatin binders, but not the EU isoform when compared to the MS2-CTL. This suggested that the exons uniquely found in NR and DQ (exons 2, 3, 4, 8, 9, 10, 11 and 12) may contain RNA domains required for chromatin recognition by ANRIL (Figure 5A). Importantly, it was shown that TEs in IncRNAs can serve for their chromatin occupancies. Thus, by using the RepeatMasker version 4.1.0 (http://www.repeatmasker.org/), we looked for such elements and found that only exons 3, 8 and 12 contained TEs as DNA element, SINE and LTR, respectively (Figure 5C). To determine which ones of these exons bind efficiently to the chromatin fraction, we performed RNA extraction from chromatin after individual overexpression of MS2-tagged exons 3, 8 and 12 in HEK293 cells. This identified only exons 3 and 8 as chromatin binders when compared to the MS2-CTL (Figure 5D). Interestingly, two LTRs belonging to the ERVL-MaLRs family were found to cover almost 70% of the 696 nts of Exon8, while repeat elements covered only 17% of the 313 nts of exon 3 (Figure 5C and Figure 6A. Note that ERVs are enriched in IncRNAs compared to SINE and LINE classes and are thought to be critical for their genomic recognition. Thus, we decided to investigate whether the presence of the ERVL-MaLR elements in Exon8 could impact ANRIL's genomic occupancy and subsequently its *trans*-activity. To test this, we engineered by the CRISPR-Cas9 approach, ANRIL gene truncated for the Exon8 in HEK293 cells, hereafter called ΔExon8 HEK293 cells. The deletion did not affect the overall expression level of ANRIL nor the CDKN2A and 2B expression (Figure 6B). However, RNA extraction from chromatin performed on ΔExon8 cells revealed a significant reduction by 60% in chromatin association of ANRIL, but not for RplpO which was used as a negative control (Figure 5E). These results strongly suggest that Exon8 containing-ERVL is responsible, at least partially, for genomic association of ANRIL.

To evaluate the global impact of the absence of Exon8 on gene expression, transcriptome analysis was performed on ΔExon8 HEK293 cells using the Clariom D microarrays from Affymetrix. Interestingly, 450 genes showed changes in expression in mutated cells when compared to the HEK293 WT (279 upregulated and 171 downregulated with an FDR <0.05, log2FC>|0.6|). As mentioned above, ANRIL's silencing activity is expected to be mediated by the recruitment of PcG to its targeted loci. Hence, we decided to focus again on the genes upregulated in the absence of Exon8. We therefore applied stringent filtering and intersected the ΔExon8 upregulated genes (n=279) with the identified ANRIL direct *trans*-targets (n=123). This revealed 9 genes fitting the criteria (*p*<5.053e-08) and that could be considered as primary targets which expression depends on ANRIL Exon8. Altogether, our data show that ANRIL's genomic recognition capacity and the expression of 9 distal loci are at least in part dependent on the presence of Exon8.

### Exon8 favors ANRIL's Association with the FIRRE and TPD52L1 Loci to Modulate their Expression through H3K27me3 Deposition

Without wishing to be bound by any particular theory, IncRNA-chromatin recognition can happen by different ways. First, through specific protein partners that serve as bridge between the DNA and the IncRNA. One of the most characterized protein involved in IncRNA/chromatin association is the heterogeneous nuclear RiboNucleoProtein U (hnRNP U) matrix protein, that is required for proper chromosomal anchoring of the Xist and FIRRE IncRNAs. By using publicly available CLIP-seq databases, we searched for evidences of direct hnRNP U binding to ANRIL's Exon8. We did not find any, suggesting that ANRIL/chromatin association via Exon8 most probably did not rely on bridging by hnRNP U. The second mechanism by which IncRNA-chromatin recognition is performed is through the direct interaction of the IncRNA with the DNA molecule via RNA-DNA hybrid duplexes formed by canonical Watson-Crick base-pairing. The resulting hybrid named R-loop has been mostly described to be responsible for regulating the expression of loci located proximally to a IncRNA-hosting gene. By using the QmRLFS R-loop predictor, we searched for potential R-loop forming sequences within the Exon8 of ANRIL, but again no hits were detected. This strongly argued for an alternative mechanism engaged by Exon8 to favor ANRIL chromatin recognition.

The recent development of computational approaches coupled to chromatin purification by RNA selection have provided evidences for an additional mechanism relying on the formation of DNA/DNA:IncRNA triple helix structures, hereafter called triplex. Triplex are formed when a single stranded RNA fragment accommodates the major groove of the double stranded DNA by Hoogsteen or reverse Hoogsteen hydrogen bonds in either parallel or anti-parallel orientation. The DNA and RNA regions involved in triplex formation are called Triplex Target Sites (TTS) and DNA Binding Domains (DBD), respectively. In order to test the hypothesis of ANRIL interaction with the chromatin via triplex formation, we used Triplex Domain Finder (TDF), a computational method which predicts triplex-forming potential between TTS and DBD based on Hoogsteen hydrogen bonds search (Kuo,C.-C. et al. ([13])). We submitted the genomic coordinates of the 3,227 ANRIL genomic binding sites against the longest ANRIL isoform NR. Strikingly, only the Exon8 was predicted to contain a significant DBD (p-value = 0.0013) (Figure 7A). The predicted Ex8-DBD had a length of 42-nts and is purine-rich as shown in the Figure 7B. It is predicted to form triplex with 422 potential DNA TTSs (13.07%) out of the 3,227 regions identified by ChIRP-seq (Figure 8A). The DBD was located within the second LTR/ERVL-MaLR element reinforcing the idea of a role of the Exon8 containing-ERVL in ANRIL's genome association (Figure 6A).

Next, to check whether TTSs were present in the 9 genes that we identified as ΔExon8 upregulated primary targets, we intersected the list of the predicted TTSs (n=422) with the list of ΔExon8 upregulated primary targets (n=9). This identified 3 genes FIRRE, TPD52L1 and LSM14A (*p*<3.999e-05), containing intronic TTSs, as being potentially targeted by ANRIL Exon8 via triplex formation. We validated by RTqPCR the significant upregulation of these 3 genes in the ΔExon8 cell line compared to the WT HEK293 cells (x4.6, x2.5 and x1.5 respectively) (Figure 7C). To gain further insight into the importance of the ANRIL Exon8 association with the FIRRE, TPD52L1 and LSM14A genes *in cellulo,* we performed ANRIL ChIRP-qPCR on the ΔExon8 HEK293 cells. We verified that the removal of Exon8 did not affect the efficiency of RNA retrieval after capturing the endogenous ANRIL from the chromatin (Figure 8B). ChIRP-qPCR analyses showed a marked dissociation of ANRIL from the FIRRE and TPD52L1 loci in the absence of Exon8, as evidenced by a 4.9- and 4.1-fold reduction, when compared to the WT HEK293 cells. LSM14A showed a smaller and not significant tendency of 2.9-fold reduction while no change was observed for the negative control (Figure 7D). These results confirm the importance of Exon8 in tethering ANRIL to two specific *trans* activated-loci probably by using its DBD to form triplex structures.

Since gene silencing of ANRIL's primary targets is presumably mediated by the recruitment of PcG proteins, we sought that the loss of Exon8 might affect H3K27me3 levels at the FIRRE, TPD52L1 and LSM14A loci. Thus, we performed ChIP-qPCR experiments using antibodies against H3K27me3 or control IgG. A reduction in ranges of 70% and 60% of H3K27me3 was observed at the promoters of FIRRE and TPD52L1, respectively, in ΔExon8 HEK293 cells compared to WT cells. No change in H3K27me3 level was observed at the LSM14A promoter nor the GAPDH locus which was used as a negative control (Figure 7E). We similarly observed a decrease in the enrichment of H3K27me3 over the distal ANRIL ChIRP-seq peaks of the FIRRE and TPD52L1 genes in ΔExon8 HEK293 cells compared to WT cells (Figure 8C). Since no effect was observed on LSM14A, we excluded this gene from our downstream analysis. Nevertheless, our data reveal that Exon8 is important for ANRIL's association with FIRRE and TPD52L1 to modulate their H3K27me3 landscape.

### Exon8 is Involved in ANRIL's Association with the FIRRE and TPD52L1 Loci Presumably through Complex DNA/DNA:RNA Structures

To investigate the triplex forming potential of Exon8 on FIRRE and TPD52L1, we tested *in cellulo* whether the transient overexpression of ANRIL Exon8 could compete with the endogenous ANRIL to form triplex and thus could neutralize the ANRIL *trans*-silencing on these genes (Figure 9A). Interestingly, a modest but statistically significant upregulation of 1.2-fold was observed for the mRNA levels of these 2 genes upon Exon8 overexpression when compared to Exon1 overexpression which was used as a negative control (Figure 9A, Figure 10A). No changes were observed for the expression of FAM83B, which was not predicted to be targeted by ANRIL via triplex formation (Figure 9A). Then, we performed a triplex capture assay on genomic DNA using an adapted protocol (Sentürk Cetin,N. ([14]). In this approach, an antisense biotinylated DNA oligo hybridizing to Exon8 was used to capture triplex formed with the full length *in vitro* transcribed Exon8 incubated with sheared genomic DNA. After recovery of triplex on streptavidin beads, associated DNA was eluted and analyzed by qPCR. Upon Exon8 pulldown with streptavidin magnetic beads, we found an efficient 2.3-fold recovery of FIRRE and TPD52L1 TTSs containing-DNA regions compared to GAPDH where no triplex was expected to be formed and that was used as a negative control. Next, using electrophoretic mobility shift assay (EMSA) as an alternative method, we tested *in vitro* the triplex forming capacity of ANRIL's Ex8-DBD (42 nts single-stranded RNA, ssRNA) with the DNA duplex (dsDNA) sequences containing the TTS associated with the selected ANRIL target genes (FIRRE and TPD52L1). As a positive control for our experiment, we used a DBD (40 nts) from the IncRNA NEAT1 which has been shown to form triplex with FLI1 dsDNA (Sentürk Cetin,N. ([14]). Upon incubation of TPD52L1 radiolabeled DNA duplex with ANRIL Ex8-DBD, a decreased electrophoretic mobility was observed on gel indicating an interaction between the DNA and the RNA (Figure 9B). A similar result was obtained with NEAT1-DBD and FLI1 dsDNA while no reduced mobility was observed with FIRRE dsDNA (Figures 10B-D). Importantly, the mobility of the formed complex was not affected upon treatment with RNase H, indicating that the observed gel shift was not due to Watson-Crick, but to Hoogsteen interactions (Figure 9B and Figure 10B). Overall, these data suggest that Exon8 contains elements required by ANRIL to modulate the expression of TPD52L1 and FIRRE loci via RNA-DNA complex formation, likely canonical triplex in the case of TDP52L1.

### DISCUSSION

The transcriptional complexity of the ANRIL locus is reflected by the production of several isoforms in a tissue specific manner. The expression of at least 3 of them positively correlate with severe pathologies such as coronary artery disease, diabetes and cancers. Therefore, they are believed to participate in disease development by inappropriate modulation of gene expression. However, the high variability in the number and identity of the regulated genes according to the model studied obscures our understanding of the mechanistic link between ANRIL and pathologies. In the present study, we provide novel information on how ANRIL negatively *trans*-regulates some genes, through identification of its direct *trans*-target genes. To circumvent the fact that ANRIL is likely to modulate the expression of many gene regulators, we combined ChIRP-seq with transcriptomic analyses. For the latter, we preferred gene expression analysis upon ANRIL knockdown in HEK293 cells, which constitutively express ANRIL compared to overexpression in cell lines which may generate experimental artifact.

We found 188 genes that we defined as direct *trans*-targets of ANRIL. Gene ontology analysis did not reveal any enriched pathways. The overlap between the genes that were previously identified upon ANRIL knockout or overexpression was low likely due to the heterogeneity in the methods and cellular models used. Nevertheless, we could identify several genes involved in cell cycle progression (CDC5L), and inflammation (IL16), pathways which are reminiscent to cancer and cardiovascular diseases linked to ANRIL. Importantly, our list of ANRIL *trans*-target genes includes non-coding genes ignored so far (SNORA14B, SNORA33, TSIX, LINC01023, LINC00923, and FIRRE). As such ncRNAs may play critical functions in cellular homeostasis, this finding opens new avenues for future investigations of ANRIL's functions, in particular in the view to better understand the connection between ANRIL and disease progression.

Interestingly, we found that 65 genes out of the 188 direct *trans-*targets experienced a lower expression upon ANRIL depletion. This observation strongly suggests a positive regulatory function of ANRIL in addition to its PcG-silencing activity. Several studies have uncovered examples of IncRNAs that can either repress or activate transcription but description of a IncRNA showing both activities is less frequently reported. For instance, HOTAIR associates with at least 2 repressive complexes, the PRC2 and CoREST complexes responsible for H3K27me3 deposition and H3K4me1-2 removal at the *HOXD* locus, respectively. In contrast, the IncRNA KHPS1 activates the expression of the enhancer RNA Sphk1 by recruiting the p300/CBP complex involved in H3K27ac deposition. In mouse, the IncRNA Fendrr modifies the chromatin signatures of genes involved in heart formation through binding to both the PRC2 and TrxG/MLL complexes leading to the deposition of H3K27me3 and H3K4me3, respectively.

We identified 123 genes directly repressed by ANRIL presumably through PcG-mediated silencing. As we found that TEs cover 35% of the ANRIL sequence, we evaluated their putative importance in ANRIL *trans-*silencing. We demonstrated that Exon8 which is 70% covered by the subcategory of LTR named ERVL-MaLR is largely involved in ANRIL genomic occupancy.

Importantly, its deletion affects the expression of 9 genes out of the 123 *trans*-targets. Since *CDKN2A* and *CDKN2B* were not found among them, we concluded that Exon8 containing-ERVL does not function in *cis* but in *trans* on a limited number of genes. This limited number of Exon8-dependent *trans*-targets emphasizes the importance of other TEs which may help ANRIL to fully act in *trans.* This also indicates that ANRIL variants are likely constituted by functional blocks and that the combination of these blocks somehow confer particular features for chromatin-linked activities. For instance, Exon8 containing-ERVL may serve for specific chromatin association, while Alu sequences would favor protein recruitment.

Recent studies suggested a potential implication of repeat elements in DNA:RNA triplex formation. Thus, we used an *in silico* predictive approach to screen for possible direct ANRIL-DNA triplex formation. Interestingly, the ERVL-MaLR in Exon8 contained a DBD predicted to form triplex with TTSs identified in 3 of the 9 genes which expression repression depends on Exon8 (the non-coding gene FIRRE, and the protein coding genes TPD52L1 and LSM14A). We showed by *in vitro* approaches that Exon8 may form triplex with at least two of these loci and confirmed the Hoogsteen base-pairing formation by EMSA only for the TPD52L1 locus. This may be explained by the fact that conditions for triplex formation *in vitro* differs from those *in cellulo* where different factors may be involved, such as nucleosomes which were shown to stabilize triplex structures. However, we could demonstrate by alternative approaches the importance of Exon8 in tethering ANRIL to these loci, since deletion of this exon was accompanied by a marked reduction in ANRIL's occupancy. Importantly, we confirmed that the down-regulation of FIRRE and TPD52L1 genes is PcG-mediated by detection of a lower H3K27me3 modification in the absence of Exon8.

FIRRE and TPD52L1 are good candidates for better understanding of how ANRIL impacts disease etiology. Indeed, TPD52L1 is a protein coding gene highly upregulated in breast cancer cell lines that was identified as a cell cycle regulator important for the completion of mitosis by interacting with 14-3-3, a negative regulator of the G2/M phase transition. Similarly, ANRIL also behaves as a cell cycle regulator by mediating the expression of tumor suppressor genes. In human, the IncRNA FIRRE which is encoded from the X chromosome is involved in post-transcriptional regulation of inflammatory genes, a pathway that is linked to ANRIL in the context of cardiovascular diseases. Upregulated in human cancer, FIRRE is considered as a marker for prognosis and diagnosis in human head and neck squamous cell carcinoma (HNSCC). In mouse, Firre was shown to regulate the nuclear architecture through distinct interchromosomal interactions with 5 genomic regions. Additional functions have been attributed to Firre such as modulating adipogenesis, key pluripotency pathways and anchoring the mouse inactive X chromosome to maintain H3K27me3 status. Even though our results display coherent links with ANRIL-linked pathways such as inflammation and cell proliferation, studies evaluating the connection between ANRIL and FIRRE/TPD52L1 in pathological situations will likely yield further mechanistic insights on the role of ANRIL's *trans*-regulatory activities in the establishment of diseases.

Finally, the pioneer ChIRP-seq experiment we performed revealed that most of the ANRIL binding sites are enriched in G/A nucleotides. This property was also observed for the HOTAIR, MEG3, TERRA and NEAT1 IncRNAs. We can speculate that such composition may favor triplex formation since G/A residues generate the most stable Hoogsteen base-pairs. This supports the emergent idea that G/A-rich sequences might serve as anchoring motifs to direct IncRNAs toward specific genomic loci. Importantly, besides its 188 *trans*-targets, ANRIL associates much widely with the genome by binding approximately 3000 sites. This may reflect the fact that, our ChIRP-seq experiments were done using tiling probes hybridizing to all ANRIL exons. Therefore, they capture as a whole, the genomic sites of the full set of ANRIL variants. Unfortunately, due to the limited abundance of some of the ANRIL isoforms, we could not evaluate their individual genomic occupancy using the dChIRP approach. We also observed that most of the ANRIL binding sites are located in non-coding areas such as introns and intergenic regions. This location is in agreement with the modulator roles of IncRNAs on enhancers activity, alternative splicing and chromatin organization. For instance, the contribution of IncRNAs on splicing was exemplified by the regulatory activity of the IncRNA asFGFR2 on the alternative splicing of the FGFR2 transcript, through the formation of a heterochromatin environment which prevents the binding of splicing factors. Remarkably, 40.3% of the ANRIL sites are intronic suggesting a possible role of ANRIL as a splicing regulator that may in part explain the gap observed between the relatively few ANRIL trans-target genes and the large number of ANRIL genomic binding sites.

### Example 2: Preparation of ANRIL-TDO (ANRIL Triplex Decoy Oligonucleotide)

Two complementary, single-stranded, unmodified oligonucleotides were synthesized and then hybridized according to the following standard protocol:

| | |
|---|---|
| - Sense oligo of sequence SEQ ID NO: 2 (100µM) | 40µL |
| - Antisense oligo of sequence SEQ ID NO: 3 (100µM) | 40µL |
| - Phusion^{®} HF buffer (5x) | 40µL |
| - H₂O | 80µL |

Incubate for 2 min at 95°C. Then, slowly reduce the temperature to 20°C.

Figure 11 shows the Hoogsteen base pairs, and misappariements.

### Example 3: Transfection of HEK293 cells by ANRIL-TDO and measurement of the expression of certain primary ANRIL targets mediated by triplex by RTqPCR

At day 1, 4 million cells per 10cm² dish (10 mL DMEM Glucose High) are inoculated and incubated overnight in the incubator at 5% CO₂ at 37°C. At day 2, the cell medium is changed. DNA/transfection reagent complexes are formed:
- Prepare MixA by adding:

| | |
|---|---|
| - Uncomplemented DMEM | 1470 uL |
| - Lipofectamine2000 (Invitrogen) Incubate for 5 min at RT | 30 µL |

- Prepare MixB by adding:

| | |
|---|---|
| - Uncomplemented DMEM | 1470 uL |
| - ANRIL-TDO1 or ANRIL-TDO2 (20µM) or H20 | 30 µL |

MixA and MixB are pulled and incubated for 20 minutes at room temperature. The mixture is then added drop by drop to the cells followed by an incubation for 5h at 37°C 5% CO2.
The cell medium is then changed and cells are incubated for 24h at 37°C 5% CO2.

At day 3, the total RNAs are extracted according to the Qiagen RNeasyKit^{®} recommendations. DNase is then used:

| | |
|---|---|
| - Total RNA | 2 µg |
| - DNAse (Roche) | 1 µL |
| - Buffer DNase10x | 2 µL |
| - Ribolock | 1 µL |
| - H20 qsp | 20 µL |

Reverse transcriptase is then performed:

| | |
|---|---|
| - ARN | 11 µL |
| - dNTP (12.5 mM) | 1 uL |
| - Hexamer (10 µM) | 1µL |

Incubation is realized during 5 minutes at 65°C, and then during 5 minutes at 4°C. To the reaction mix are added:

| | |
|---|---|
| - Buffer 5x SSIIIRT | 4 µL |
| - DTT (10mM) | 1 µL |
| - Ribolock^{®} | 1 µL |
| - SuperScript III Reverse Transcriptase | 1 µL |

Incubation is performed 5 minutes at 25°C, then during 45 minutes at 50°C and finally during 15 minutes at 70°C. 30 uL of H2O are added, and 1 µL of the mixture is used for qPCR reactions.

Results are presented in Figure 12. Main comments and conclusions are described below
1/ A trend of the targeted genes (except for LSM14A) to have higher expression level after treatment is observed. This is expected in case of an active TDO since ANRIL silences the targeted genes.
2/ ANRIL level does not seem to be affected by the TDO treatment. This is coherent with our hypothesis that TDF should not affect ANRIL stability but acts on its ability to associate with chromatin via triplex formation.
3/ The effect on gene expression seems to be better with ANRIL-TDO2.
4/ The increased level of the genes of interest does not exceed 2-fold (except for KRTDAP, no obvious reason yet). This apparent weak effect is in fact expected since our previous data suggest that depletion of ANRIL's Exon8 affects slighly but significantly (from 1.5x to 3.8x depending on the considered genes) expression of the targeted genes.

### Reference List

1. Rajagopal, P., and J. Feigon: "Triple-Strand Formation in the Homopurine:homopyrimidine DNA Oligonucleotides d(G-A)4 and d(T-C)4." Nature 339, no. 6226 (June 22, 1989): 637-40.
2. Maldonado et al.: "Purine- and Pyrimidine-Triple-Helix-Forming Oligonucleotides Recognize Qualitatively Different Target Sites at the Ribosomal DNA Locus." RNA (New York, N.Y.) 24, no. 3 (2018): 371-80.
3. Crinelli, R. et al.: "Locked Nucleic Acids (LNA): Versatile Tools for Designing Oligonucleotide Decoys with High Stability and Affinity." Current Drug Targets 5, no. 8 (November 2004): 745-52.
4. Hecker, Markus, and Andreas H. Wagner: "Transcription Factor Decoy Technology: A Therapeutic Update." Biochemical Pharmacology 144 (15 2017): 29-34.
5. Ran,F.A. et al.: "Genome engineering using the CRISPR-Cas9 system". Nat. Protoc., 8 (2013), 2281-2308.
6. Chu,C. et al.: "Genomic maps of lincRNA occupancy reveal principles of RNA-chromatin interactions". Mol. Cell, 44 (2011), 667-678.
7. Langmead,B. and Salzberg,S.L.: "Fast gapped-read alignment with Bowtie 2". Nat. Methods, 9 (2012), 357-359.
8. Quinlan,A.R. and Hall,I.M.: "BEDTools: a flexible suite of utilities for comparing genomic features". Bioinformatics, 26 (2010), 841-842.
9. Zhang,Y. et al.: "Model-based Analysis of ChIP-Seq (MACS)". Genome Biol., 9 (2008), R137.
10 Yu,G., Wang,L.-G. and He,Q.-Y.: "ChIPseeker: an R/Bioconductor package for ChIP peak annotation, comparison and visualization". Bioinformatics, 31 (2015), 2382-2383.
11. Bailey,T.L. and Elkan,C.: "Fitting a Mixture Model By Expectation Maximization To Discover Motifs In Biopolymer". Proc. Int. Conf. Intell. Syst. Mol. Biol., 2 (1994), 28-36.
12. Bailey,T.L. et al.: "MEME SUITE: tools for motif discovery and searching". Nucleic Acids Res., 37 (2009), W202-W208.
13. Kuo,C.-C. et al.: "Detection of RNA-DNA binding sites in long noncoding RNAs". Nucleic Acids Res., 47 (2019), e32-e32.
14. Sentürk Cetin,N. et al.: "Isolation and genome-wide characterization of cellular DNA:RNA triplex structures". Nucleic Acids Res., 47 (2019), 2306-2321.
15. Engreitz,J.M. et al.: "The Xist IncRNA exploits three-dimensional genome architecture to spread across the X-chromosome". Science, 341 (2013), 1237973.
16. Jeon,Y. and Lee,J.T.: "YY1 tethers Xist RNA to the inactive X nucleation center". Cell, 146 (2011), 119-133.
17. Kotake,Y. et al.: "Long non-coding RNA ANRIL is required for the PRC2 recruitment to and silencing of p15INK4B tumor suppressor gene". Oncogene, 30 (2011), 1956-1962.
18. Wardwell et al.: "Immunomodulation of cystic fibrosis epithelial cells via NF-κB decoy oligonucleotide-coated polysaccharide nanoparticles", J Biomed Mater Res A., 2015 May; 103(5): 1622-31.
19. Farahmand et al.: "Suppression of chronic inflammation with engineered nanomaterials delivering nuclear factor κB transcription factor decoy oligodeoxynucleotides", Drug. Deliv.. 2017 Nov;24(1):1249-1261.
20. Mamet et al.: "Pharmacology, pharmacokinetics, and metabolism of the DNA-decoy AYX1 for the prevention of acute and chronic post-surgical pain", Mol Pain. 2017 Jan;13:1744806917703112.

## Claims

1. An isolated double stranded DNA polynucleotide that forms triplex with sequence
5'-GGUGGCAGCAAGAGAAAAAUGAGGAAGAAGCAAAAGCGGAAA-3' (SEQ ID NO: 1) of the long non-coding RNA ANRIL (Antisense Non-coding RNA in the INK4 Locus).

2. An isolated double stranded DNA polynucleotide according to claim 1, which has a sense oligonucleotide having at least 85% sequence identity with sequence
5'-AAAGGCGAAAACGAAGAAGGAGTAAAAAGAGAACGACGGTGG-3' (SEQ ID NO: 2)

3. An isolated double stranded DNA polynucleotide according to claim 1, which has an antisense oligonucleotide having at least 85% sequence identity with sequence
5'-CCACCGTCGTTCTCTTTTTACTCCTTCTTCGTTTTCGCCTTT-3' (SEQ ID NO: 3).

4. An isolated double stranded DNA polynucleotide according to anyone of the preceding claims, which has:
- a sense oligonucleotide consisting of sequence 5'-AAAGGCGAAAACGAAGAAGGAGTAAAAAGAGAACGACGGTGG-3' (SEQ ID NO: 2), and
- an antisense oligonucleotide consisting of sequence 5'-CCACCGTCGTTCTCTTTTTACTCCTTCTTCGTTTTCGCCTTT-3' (SEQ ID NO: 3).

5. An isolated double stranded DNA polynucleotide according to anyone of claims 1 to 3, which has:
- a sense oligonucleotide consisting of sequence 5'-AAAGGGGAAAAGGAAGAAGGAGAAAAAAGAGAAGGAGGGAGG-3' (SEQ ID NO: 4), and/or
- an antisense oligonucleotide consisting of sequence 5'-CCTCCCTCCTTCTCTTTTTTCTCCTTCTTCCTTTTCCCCTTT-3' (SEQ ID NO: 5).

6. An isolated double stranded DNA polynucleotide according to anyone of the preceding claims, comprising at least one modification chosen among biostability-enhancing chemical modifications such as locked nucleic acids and/or phosphorothioate bonds.

7. A vector comprising a double stranded DNA polynucleotide as defined in anyone of the preceding claims.

8. A vector according to claim 7, **characterized in that** it is chosen among polymers, such as poly (D,L-lactide co-glicolide) or chitosan, liposomes, gelatin, lipid based nanoparticles, viruses, such as adenoviruses, adeno-associated viruses or retroviruses, and antibodies.

9. A pharmaceutical composition comprising a double stranded DNA polynucleotide as defined in claims 1 to 6, or a vector as defined in anyone of claims 7 or 8.

10. Use of sequence SEQ ID NO : 1 of ANRIL in a method of preparation of a double stranded DNA polynucleotide as defined in claims 1 to 6.

11. A method of preparation of a double stranded DNA polynucleotide as defined in claims 1 to 6, comprising a step of synthesizing or isolating a double stranded DNA polynucleotide forming triplex with sequence SEQ ID NO : 1 of ANRIL.

12. An isolated double stranded DNA polynucleotide as defined in anyone of claims 1 to 6, for use in the treatment of myocardial infarction, aneurysms, stenosis, myocardial infarction, aneurysms, cancers, eye diseases or type 2 diabetes.
